# EUROPEAN PATENT APPLICATION

(11) **EP 4 834 887 A1**
(43) Date of publication of application: **04.11.2026**
(21) Application number: 24913014.7
(22) Date of filing: 26.12.2024
(51) Int. Cl.: C07D 251/34, C08G 59/48

(54) **REACTANT, CURABLE COMPOSITION, AND REACTION METHOD OF EPOXY COMPOUND**

(30) Priority: 28.12.2023 JP 2023223593
(71) Applicant: Kureha Corporation, Chuo-ku Tokyo 103-8552 (JP)
(72) Inventor: KUDO, Takahiro, Tokyo 103-8552 (JP); TAJIMA, Saori, Tokyo 103-8552 (JP); ISHIDA, Naoyoshi, Tokyo 103-8552 (JP); FUJII, Kenshiro, Tokyo 103-8552 (JP)
(74) Representative: Hoefer & Partner Patentanwälte mbB
(86) International application number: PCT/JP2024/046169
(87) International publication number: WO 2025/143128

(57) **Abstract**

A reactant for reacting with an epoxy compound, the reactant including an active ester compound having a structure in which an acyloxy group is bonded to a carbon atom constituting a 1,3,5-triazine ring.

## Description

### TECHNICAL FIELD

The present invention relates to a reactant, a curable composition, and a method of reacting an epoxy compound.

### BACKGROUND ART

Epoxy compounds exhibit high reactivity and are therefore used as intermediates for various reactions and as raw materials for introducing functional groups. Epoxy compounds are also used as materials that provide cured products by reaction with curing agents.

For example, as a curing reaction of an epoxy compound, a method of reacting an epoxy compound with a compound having active hydrogen, such as an amine-based curing agent, is known. In the reaction with the compound having active hydrogen, for example, an active hydrogen group such as an amino group is added to an epoxy group of an epoxy compound, lead to ring-opening of the epoxy group to form a hydroxy group. Thus, the hygroscopicity and dielectric constant of the resulting cured product may increase in some cases.

In view of this, a method of reacting an epoxy compound with an active ester compound has been studied. In this reaction, since no hydroxy group is formed after ring-opening of the epoxy group of the epoxy compound, increases in the hygroscopicity and dielectric constant of the resulting cured product can be suppressed. As the active ester compound, benzyl acetate, p-substituted phenyl acetates, methyl benzoate, benzyl thioacetate, and the like are known (see Non-Patent Literature 1).

### CITATION LIST

### NON-PATENT LITERATURE

Non-Patent Literature 1: Nippon Kagaku Kaishi, 1991, No. 11, Addition reactions between cyclic ethers and various esters (Kanjo eteru-rui to shuju no esuteru to no fuka hannou)

### SUMMARY OF INVENTION

### TECHNICAL PROBLEM

However, known active ester compounds still lack sufficient reactivity with epoxy compounds. A high reaction temperature is therefore needed for reaction with epoxy compounds.

The present invention has been made in view of the above circumstances, and provides a reactant that reacts with an epoxy compound at a low reaction temperature and can suppress the formation of a hydroxy group, a curable composition, and a method of reacting an epoxy compound.

### SOLUTION TO PROBLEM

[1] A reactant for reacting with an epoxy compound, the reactant being an active ester compound having a structure in which an acyloxy group is directly bonded to a carbon atom constituting a 1,3,5-triazine ring.
[2] The reactant according to [1], wherein
   the reactant is a modifying agent for reacting with an epoxy compound; and
   the reactant is a compound represented by Formula (1) below: 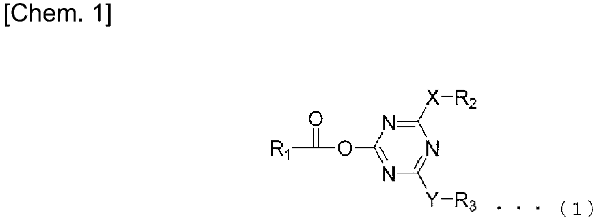
   wherein in Formula (1),
   R₁ to R₃ are each a monovalent organic group; and
   X and Y are each an oxygen atom, -NR- (where R is a substituent), or a sulfur atom.
[3] The reactant according to [1], wherein
   the reactant is a curing agent for an epoxy compound; and
   the active ester compound is a compound represented by Formula (2) below: 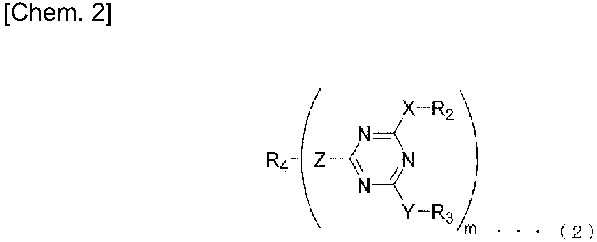
   wherein in Formula (2),
   R₂ and R₃ are each a monovalent organic group;
   R₄ is an m-valent organic group;
   m is an integer of 1 or greater;
   when m is 1, at least two of X, Y, or Z are carbonyloxy bonds, and the remainder is an oxygen atom, -NR- (where R is a substituent), or a sulfur atom; and
   when m is 2 or greater, at least one of X, Y, or Z is a carbonyloxy bond, and the remainder is an oxygen atom, -NR- (where R is a substituent), or a sulfur atom.
[4] The reactant according to [3], wherein
   m is an integer of 2 or greater;
   Z is a carbonyloxy bond; and
   X and Y are each an oxygen atom, -NR-, or a sulfur atom.
[5] The reactant according to [3], wherein
   m is an integer of 2 or greater;
   X and Y are each a carbonyloxy bond; and
   Z is an oxygen atom, -NR-, or a sulfur atom.
[6] The reactant according to [1], wherein
   the reactant is a curing agent for an epoxy compound; and
   the active ester compound is a polymer having a structure represented by any one of Formulae (3) to (6) below: 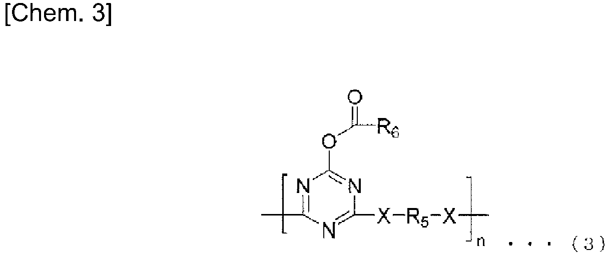 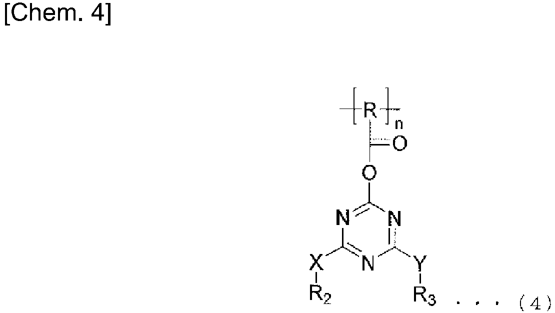
   wherein in Formulae (3) and (4),
   R₂, R₃, and R₆ are each a monovalent organic group;
   R₅ is a divalent organic group;
   R is a group constituting a repeating unit of a polymer;
   X and Y are each an oxygen atom, -NR- (where R is a substituent), or a sulfur atom; and
   n is an integer of 2 or greater, and 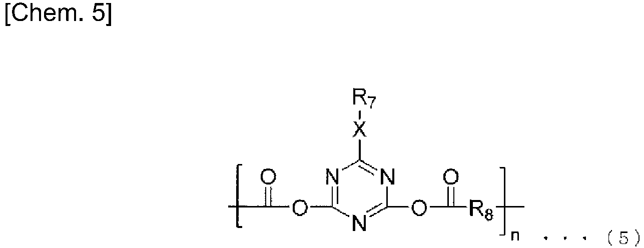 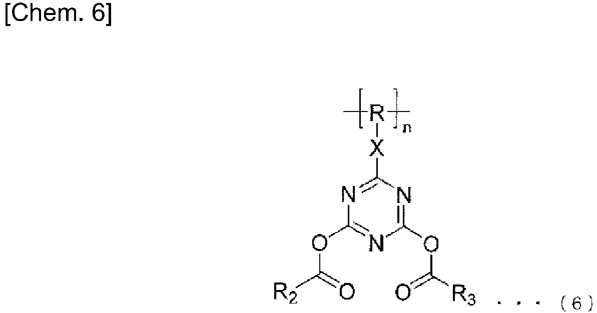
   wherein in Formulae (5) and (6),
   R₂, R₃, and R₇ are each a monovalent organic group;
   R₈ is a divalent organic group;
   R is a group constituting a repeating unit of a polymer;
   X is an oxygen atom, -NR- (where R is a substituent), or a sulfur atom; and
   n is an integer of 2 or greater.
[7] A curable composition, including:
   an epoxy compound; and
   the reactant described in any one of [3] to [6].
[8] The curable composition according to [7], wherein
   the epoxy compound is an alicyclic epoxy compound.
[9] A method of reacting an epoxy compound, the method including:
   reacting an epoxy compound with the reactant described in any one of [1] to [6].
[10] The reaction method according to [9], wherein the epoxy compound is an alicyclic epoxy compound.

### ADVANTAGEOUS EFFECTS OF INVENTION

According to the present invention, it is possible to provide a reactant, a curable composition, and a method of reacting an epoxy compound, which are capable of reacting with an epoxy compound at a low reaction temperature and suppressing the formation of a hydroxy group.

### BRIEF DESCRIPTION OF DRAWINGS

FIG. 1 is a graph showing the results of DSC measurement of a monofunctional active ester compound.
FIG. 2 is a graph showing the results of DSC measurement of a multifunctional active ester compound.
FIG. 3A is a graph showing the results of DSC measurement of Compound F, and FIG. 3B is a graph showing the results of DSC measurement of Compound H.
FIG. 4 is a graph showing the results of DSC measurement of TAB.
FIG. 5 is a graph showing the results of DSC measurement of Compound H with different types of epoxy compounds.
FIG. 6 illustrates photographs of the state of each curable composition after drying, taken from directly above the bottom of a vial.

### DESCRIPTION OF EMBODIMENTS

The present inventors have found that an active ester compound having a structure in which an acyloxy group is directly bonded to a carbon atom of a triazine ring exhibits high reactivity with an epoxy compound unlike known active ester compounds, and specifically, the active ester compound undergoes an addition reaction with an epoxy group of an epoxy compound even at a low reaction temperature.

That is, one embodiment of the present invention relates to a reactant for reacting with an epoxy group. The reactant is a reactant for reacting with an epoxy compound, and includes an active ester compound having an acyloxy group bonded to a carbon atom constituting a 1,3,5-triazine ring. The reactant preferably includes an active ester compound having a structure in which an acyloxy group is directly bonded to a carbon atom constituting a 1,3,5-triazine ring in one molecule.

The acyloxy group (R-CO-O-) refers to a functional group in which an acyl group (R-CO-) and an oxy group (-O-) are bonded (where R in R-CO-O- and R-CO- both represent a substituent). The structure in which an acyloxy group is directly bonded to a carbon atom constituting a 1,3,5-triazine ring is represented by, for example, R-CO-O-* (where * represents a site bonded to a carbon atom constituting the triazine ring).
The number of acyloxy groups directly bonded to a carbon atom constituting the 1,3,5-triazine ring in one molecule of the active ester compound may be one (monofunctional) or two or more (multifunctional).

The active ester compound having such a structure can be added to an epoxy group of an epoxy compound even at a low reaction temperature, and can suppress the formation of a hydroxy group in the reaction product. Therefore, the active ester compound having the above structure can be used as a reactant, for example, a modifying agent for introducing various functional groups by reacting with an epoxy group, or can be used as a curing agent for crosslinking an epoxy compound.

In the present specification, the term "reactant" refers to a chemical substance that reacts with an epoxy group of an epoxy compound to form a chemical bond. An amine compound, an alcohol compound, a phenol compound, and a thiol compound react with an epoxy group in a nucleophilic manner, whereas the above active ester compound reacts in an electrophilic manner similarly to an acid anhydride and an isocyanate.

The epoxy compound to be reacted with the reactant is not particularly limited as long as it is a compound containing an epoxy group in its molecule. The epoxy compound may be a compound having a glycidyl group or a compound having an alicyclic epoxy structure. For example, an alicyclic epoxy compound is particularly preferable from the viewpoint of being capable of reacting with the above reactant at a lower temperature.

Hereinafter, the case of using the reactant as a modifying agent or a curing agent will be described. Note that the expression "to" in the present specification means a numerical range including the lower limit and the upper limit as end points, unless otherwise specified.

### First Embodiment

### 1. Modifying Agent

One embodiment of the present invention relates to a chemical substance for reacting with an epoxy group, and the chemical substance is referred to as a modifying agent. The modifying agent refers to a chemical substance used for introducing various functional groups into an epoxy compound by chemically bonding to an epoxy group of an epoxy compound, thereby altering the properties of the epoxy compound. The modifying agent includes an active ester compound having a structure in which an acyloxy group is bonded to a carbon atom constituting a 1,3,5-triazine ring in one molecule. For example, the active ester compound is preferably a compound represented by Formula (1) below:

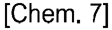

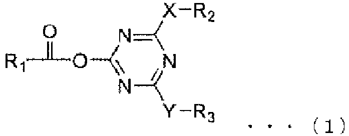

In Formula (1), R₁ to R₃ are each a monovalent organic group. The monovalent organic group is a monovalent aliphatic group or a monovalent aromatic group, and the monovalent aliphatic group or the monovalent aromatic group may contain another element in addition to C and H, such as N, O, F, Cl, Br, S, P, or Si.

The monovalent aliphatic group that may contain another element means a residue obtained by removing one hydrogen atom from a carbon atom in an aliphatic compound. The above aliphatic compound includes a compound in which a plurality of aliphatic chains are bonded via a linking group such as an ether bond, and also includes a saturated heterocyclic ring.
That is, the monovalent aliphatic group may be linear, branched, or cyclic, and includes not only a residue consisting of C and H but also a residue containing an element in addition to C and H. Examples of the monovalent aliphatic group include linear alkyl groups having from 1 to 18 carbons such as a methyl group, an ethyl group, and a propyl group; branched alkyl groups having from 3 to 10 carbons such as an isopropyl group, a sec-butyl group, and an isobutyl group; cycloalkyl groups having from 3 to 10 carbons such as a cyclohexyl group; and heterocyclic groups such as tetrahydrofuran. These monovalent aliphatic groups may further have a substituent such as an aryl group or a halogen atom.

The monovalent aromatic group that may contain another element means a residue obtained by removing one hydrogen atom from a carbon atom of an aromatic ring in an aromatic compound. The above aromatic compound also includes a compound in which a plurality of aromatic rings are bonded or condensed, such as biphenyl and naphthalene.
That is, the monovalent aromatic group includes not only a residue consisting of C and H but also a residue containing an element in addition to C and H. The number of carbons of the monovalent aromatic group may be, for example, from 5 to 20. Examples of the above aromatic group include aryl groups such as a phenyl group and a naphthalene group; and heteroaryl groups such as a pyridinyl group. These monovalent aromatic groups may further have a substituent such as an alkyl group, an alkoxy group, or a halogen atom.

Among these, R₁ to R₃ are each preferably an alkyl group or an aryl group, and more preferably an alkyl group having 3 or less carbons such as a methyl group, an ethyl group, or a propyl group, or a phenyl group. Among these, R₁ is preferably a phenyl group. Furthermore, R₂ and R₃ are preferably an alkyl group having 3 or less carbons such as a methyl group, an ethyl group, or a propyl group, and particularly preferably a methyl group.

In Formula (1), X and Y are each an oxygen atom, -NR-, or a sulfur atom. Among these, an oxygen atom or a sulfur atom is preferable, and an oxygen atom is more preferable. R in -NR- is a hydrogen atom or a substituent, and is preferably a hydrogen atom, an alkyl group having from 1 to 4 carbons, or an aryl group having from 6 to 10 carbons. When R is an alkyl group, R and R₂ or R₃ may be bonded to each other to form a ring (for example, a nitrogen-containing aliphatic six-membered ring).

As preferable examples of the compound represented by Formula (1), preferable is a compound in which R₁ is an aliphatic group or an aryl group, R₂ and R₃ are each an alkyl group or an aryl group, and X and Y are oxygen atoms or sulfur atoms; more preferable is a compound in which R₁ is a cycloalkyl group or a phenyl group, R₂ and R₃ are each an alkyl group having 3 or less carbons such as a methyl group, an ethyl group, or a propyl group, or a phenyl group, and X and Y are oxygen atoms; still more preferable is a compound in which R₁ is a phenyl group, R₂ and R₃ are alkyl groups having 3 or less carbons such as a methyl group, an ethyl group, or a propyl group, and X and Y are oxygen atoms; and particularly preferable is a compound in which R₁ is a phenyl group, R₂ and R₃ are methyl groups, and X and Y are oxygen atoms.

Specific examples of the compound represented by Formula (1) include the following compounds.

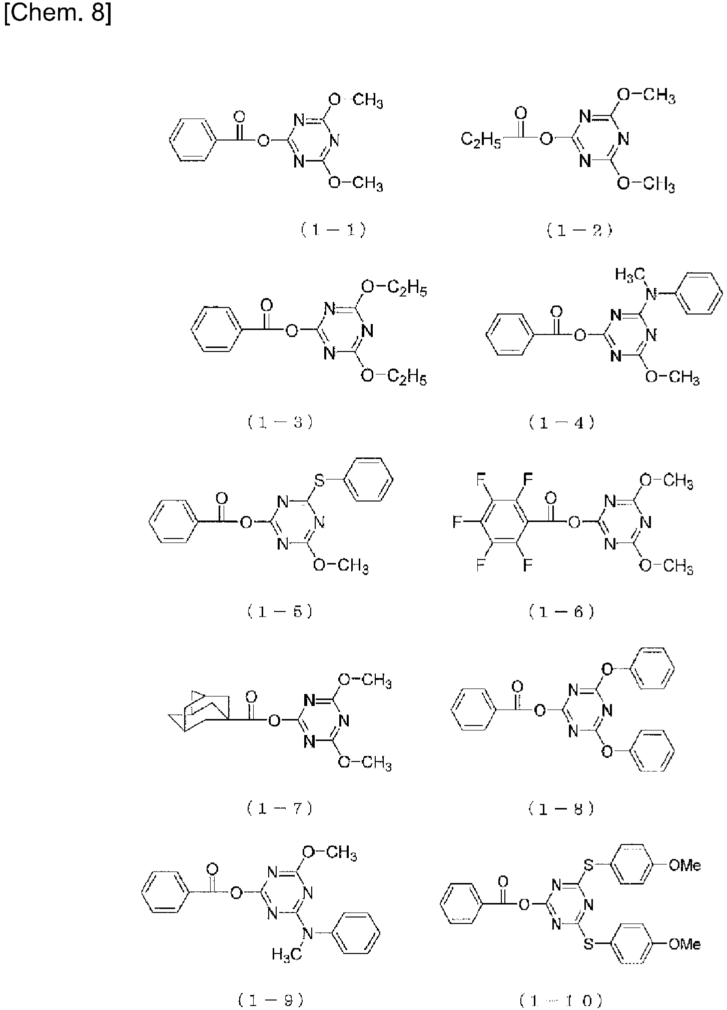

### 2. Method of Reacting Epoxy Compound

As described above, the compound represented by Formula (1) is an active ester compound, and an ester group constituting the active ester compound is added to a ring-opened epoxy group of an epoxy compound. The type of epoxy compound is not particularly limited, and an epoxy compound described later can be used. In addition, any functional groups different from each other can be introduced at the 2-, 4-, and 6-positions of the triazine ring in the active ester compound. Therefore, the above compound can be utilized as a modifying agent for introducing a new functional group into an epoxy compound, or the like.

For example, A. W. C. Lin et al., J. Electroanal. Chem., 84, pp411-419 (1977) and Sawako Shibata et al., Sen'i Gakkaishi, vol. 69, No. 12 (2013) have reported a method of imparting functionalities and new physical properties to material surfaces by reacting active hydrogen-containing functional groups present on the material surfaces with cyanuric chloride and then substituting the remaining chloride atoms with the functional groups.

As a specific example of utilizing a triazine compound as a modifying agent (functional compound) for introducing a new functional group, a chlorotriazine compound having cyclodextrin has been reported, and a material to which the inclusion ability of cyclodextrin is imparted by modifying a fiber with the compound is known (for example, see JP 2002-65839 A).
In addition, the functional compound is not limited, and a reaction product of bis (2,2,6,6-tetramethyl-4-piperidyl)amine and cyanuric chloride, which imparts a function as an oxidation inhibitor (JP 63-69203), a reaction product of acetoxime and cyanuric chloride, which imparts a radical generating function (JP 2015-538399), and a reaction product of aminocyanine dye and cyanuric chloride, which imparts a fluorescent function (JP 9-104825 A) have been reported.

The active ester compound in the present embodiment also has various functional groups as substituents (-X-R₂ and -Y-R₃ in Formula (1)) other than the acyloxy group bonded to the triazine ring. Accordingly, the acyloxy group of the active ester compound is reacted with the epoxy group of the epoxy compound, thereby making it possible to suppress the formation of active hydrogen inside or on the surface of the epoxy compound. That is, the functional group can be introduced while suppressing the formation of a hydroxy group.

The following reaction formula illustrates a reaction example using 2,4-dimethoxy-6-benzoyloxy-1,3,5-triazine as the compound represented by Formula (1) and glycidyl phenyl ether (GPE) as the epoxy compound. As illustrated in the following reaction formula, when the compound represented by Formula (1) is added to an epoxy group, an oxygen atom of an epoxy ring forms an ester. Therefore, unlike the case of reacting with a functional group having active hydrogen, such as amines, no hydroxy group is formed. This makes it possible to impart a function derived from the triazine ring or a functional group bonded thereto without increasing the hygroscopicity or dielectric constant of a reaction product.

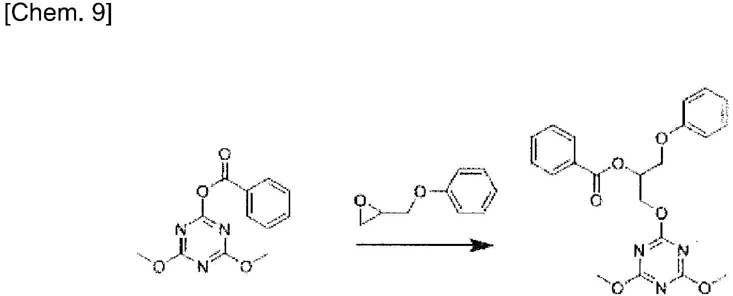

### Second Embodiment

### 1. Curing Agent

One embodiment of the present invention relates to a curing agent for crosslinking an epoxy compound. The curing agent includes an active ester compound (multifunctional active ester compound) having two or more acyloxy groups directly bonded to carbon atoms constituting a 1,3,5-triazine ring in one molecule. The multifunctional active ester compound can react with an epoxy compound at a still lower reaction temperature than that for the above monofunctional active ester compound. The active ester compound may be a low molecular weight compound or a high molecular weight compound.

### 1-1. Low Molecular Weight Compound

The active ester compound is preferably a compound represented by Formula (2) below.

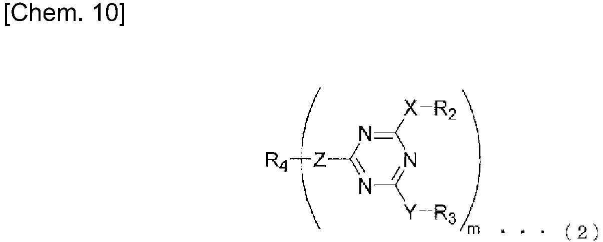

R₂ and R₃ in Formula (2) have the same meanings as R₂ and R₃ in Formula (1), respectively. When m is an integer of 2 or greater, a plurality of R₂ may be mutually the same or different. Furthermore, a plurality of R₃ may be mutually the same or different.

R₄ in Formula (2) is an m-valent organic group. The m-valent organic group is an m-valent aliphatic group or an m-valent aromatic group, and the m-valent aliphatic group or the m-valent aromatic group may contain another element in addition to C and H, such as N, O, F, Cl, Br, S, P, or Si.

The m-valent aliphatic group that may contain another element means a residue obtained by removing m hydrogen atoms from different carbon atoms of an aliphatic compound, and the above aliphatic compound also includes a compound in which a plurality of aliphatic chains are bonded via a linking group such as an ether bond, and a saturated heterocyclic ring.
That is, the m-valent aliphatic group may be linear, branched, or cyclic, and includes not only a residue consisting of C and H but also a residue containing an element other than C and H. Examples of the m-valent aliphatic group include residues of linear alkyl having from 1 to 18 carbons such as a methylene group, an ethylene group, a propylene group, a butylene group, a pentylene group, a hexylene group, a heptylene group, and an octylene group; residues of branched alkyl having from 3 to 10 carbons such as an isopropylene group, sec-butylene, and an isobutylene group; residues of cycloalkyl having from 6 to 8 carbons such as a cyclohexylene group; and residues of heterocycle such as a piperazinyl group. These m-valent aliphatic groups may further have a substituent described above.

The m-valent aromatic group that may contain another element means a residue obtained by removing m hydrogen atoms from different carbon atoms of an aromatic ring in an aromatic compound, and the above aromatic compound also includes a compound in which a plurality of aromatic rings are bonded or condensed, such as biphenyl or naphthalene.
That is, the m-valent aromatic group includes not only a residue consisting of C and H but also a residue containing an element other than C and H. Examples of the m-valent aromatic group include residues of benzene, biphenyl, diphenyl ether, diphenyl sulfone, diphenyl ketone, and naphthalene; and residues of heterocyclic compounds such as pyridine and pyrimidine. These m-valent aromatics may further have a substituent described above.

From the viewpoint of further increasing the reactivity, R₄ is preferably an aromatic group. Although depending on the combination with R₂ or R₃, the aromatic group is more preferably an aromatic group optionally having an electron-withdrawing group, particularly preferably a group derived from an aromatic ring selected from the group consisting of benzene, naphthalene, diphenyl ether, hexafluoro-2,2-diphenylpropane, benzenesulfonic acid, chlorobenzene, bromobenzene, and iodobenzene, and most preferably a group derived from an aromatic ring selected from the group consisting of diphenyl ether and hexafluoro-2,2-diphenylpropane.

In Formula (2), m is an integer of 1 or greater. Among these, from the viewpoint of further enhancing reactivity with an epoxy compound, m is preferably 2 or greater, and more preferably 3 or greater. The upper limit value of m is, for example, 6.

When m is 1, at least two of X, Y, or Z are carbonyloxy bonds, and the remainder is an oxygen atom, -NR-, or a sulfur atom, preferably an oxygen atom or a sulfur atom, and more preferably an oxygen atom. All of X, Y and Z may be carbonyloxy bonds.
When m is 2 or greater, at least one of X, Y, or Z is a carbonyloxy bond, and the remainder is an oxygen atom, -NR-, or a sulfur atom, preferably an oxygen atom or a sulfur atom, and more preferably an oxygen atom.
Note that the carbonyloxy bond is represented by *-O-C(=O)- (where * represents a site bonded to a carbon atom constituting the triazine ring).

Furthermore, R in -NR- is a hydrogen atom or a substituent, and is preferably a hydrogen atom, an alkyl group having from 1 to 4 carbons, or an aryl group having from 6 to 10 carbons. When R is an alkyl group, R and R₂, R₃, or R₄ may be bonded to each other to form a ring (for example, a nitrogen-containing aliphatic six-membered ring).
In addition, in a case where m is 2 or greater, when R₄ is an m-valent aliphatic group (for example, an alkyl group) and Z is -NR-, R of two -NR- may be bonded to each other to form a ring (for example, a nitrogen-containing aliphatic six-membered ring). For example, in a case where m is 2, when R₄ is an alkylene group and Z is -NR-, -Z-R₄-Z- in Formula (2) may form a piperazinyl ring represented by the following formula.

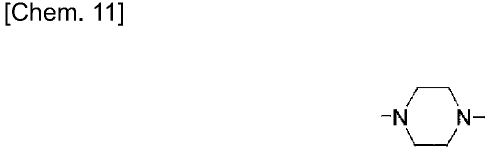

When m is 2 or greater, for example, X may be a carbonyloxy bond, and Y and Z may each be an oxygen atom, -NR-, or a sulfur atom. Alternatively, X and Y may each be an oxygen atom, -NR-, or a sulfur atom, and Z may be a carbonyloxy bond (see Formula (2A)). Alternatively, X and Y may each be a carbonyloxy bond, and Z may be an oxygen atom, -NR-, or a sulfur atom (see Formula (2B)).

That is, the active ester compound may be a compound having a plurality of structures in which one acyloxy group is bonded to a triazine ring in its molecule (low molecular weight compound A), or may be a compound having a plurality of structures in which two acyloxy groups are bonded to a triazine ring in its molecule (low molecular weight compound B).

### 1-1-1. Low Molecular Weight Compound A

The compound represented by Formula (2) may be a compound represented by Formula (2A):

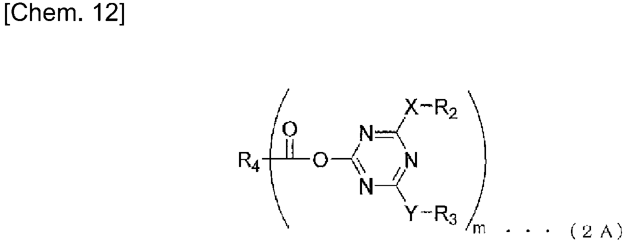

R₂ to R₄, X, and Y in Formula (2A) have the same meanings as R₂ to R₄, X, and Y in Formula (2), respectively. When m is an integer of 2 or greater, a plurality of R₂ may be mutually the same or different. Furthermore, a plurality of R₃ may be mutually the same or different.

m in Formula (2A) is an integer of 2 or greater. From the viewpoint of further enhancing the curability of an epoxy compound, m is preferably 2 or greater, and m is more preferably 3 or greater. The upper limit value of m is not particularly limited and can be, for example, 6 or less.

As preferable examples of the compound represented by Formula (2A), preferable is a compound in which m is an integer from 2 to 4, R₄ is an aromatic group, R₂ and R₃ are each an alkyl group or an aryl group, and X and Y are oxygen atoms or sulfur atoms; more preferable is a compound in which m is an integer from 2 to 3, R₄ is an aromatic group, R₂ and R₃ are each an alkyl group having 3 or less carbons such as a methyl group, an ethyl group, or a propyl group, or a phenyl group, and X and Y are oxygen atoms; still more preferable is a compound in which m is an integer from 2 to 3, R₄ is a group derived from a benzene ring, R₂ and R₃ are alkyl groups having 3 or less carbons such as methyl groups, ethyl groups, or propyl groups, and X and Y are oxygen atoms; particularly preferable is a compound in which m is 3, R₄ is a group derived from a benzene ring, R₃ and R₄ are methyl groups, and X and Y are oxygen atoms.

Specific examples of the compound represented by Formula (2A) include the following compounds.

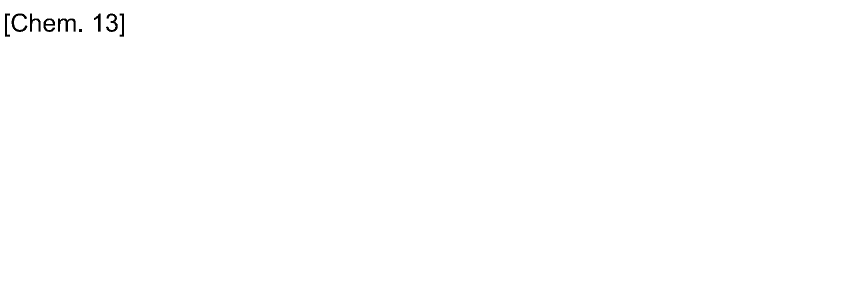

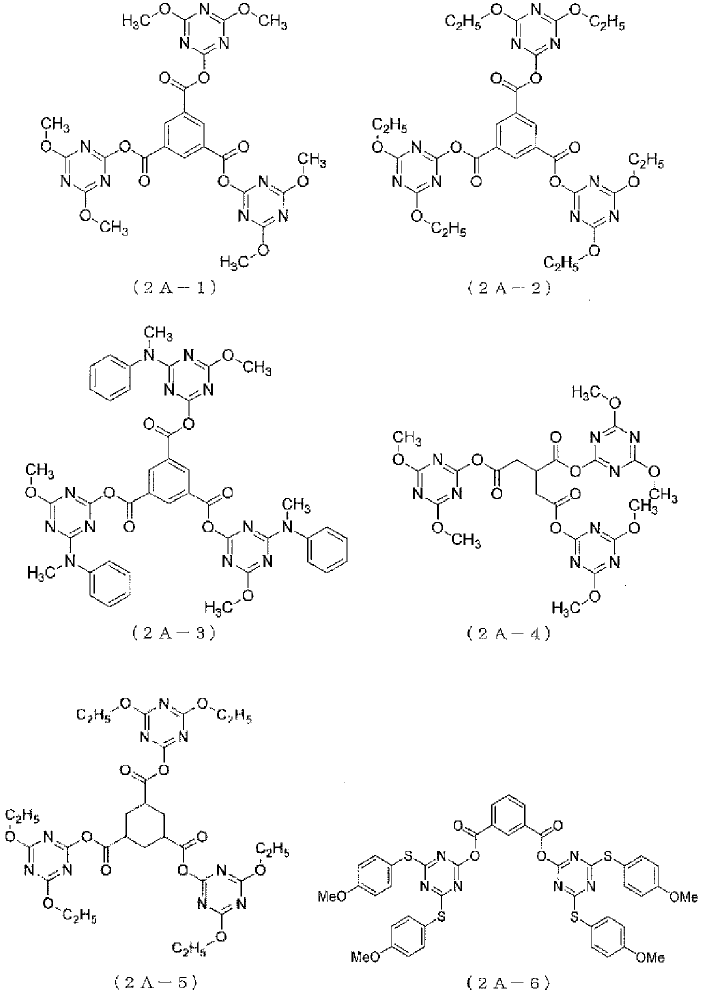

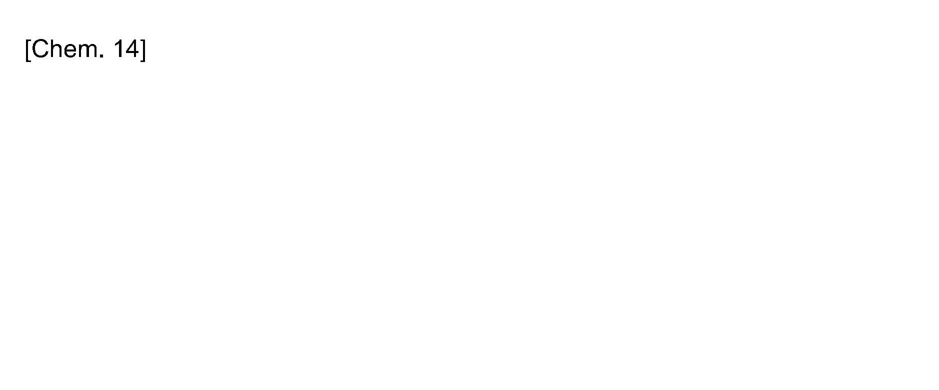

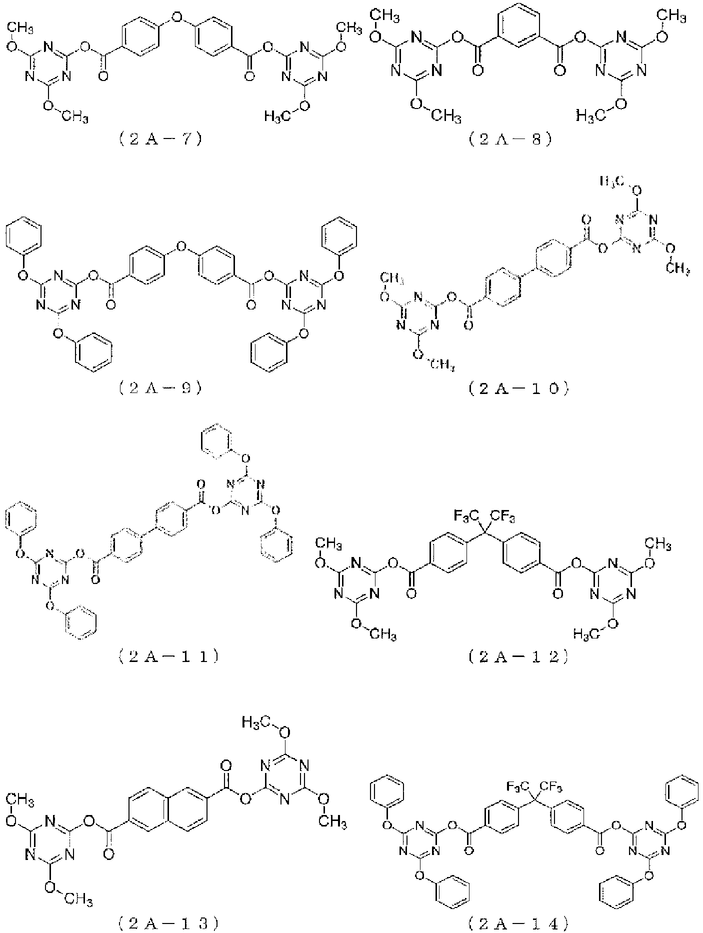

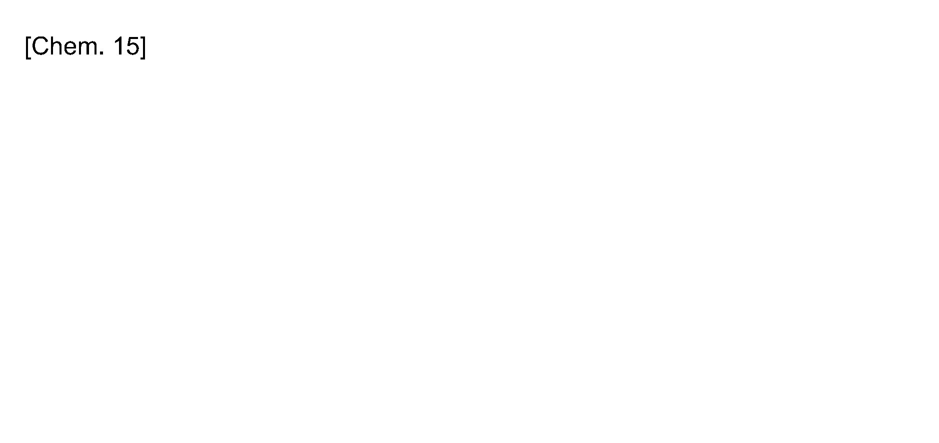

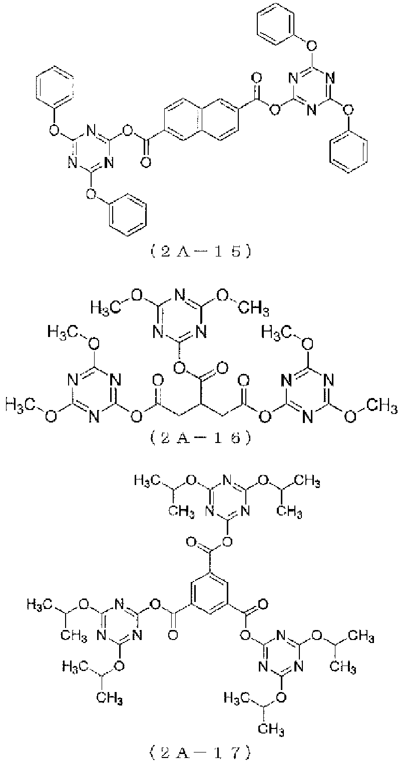

### 1-1-2. Low Molecular Weight Compound B

The compound represented by Formula (2) may be a compound represented by Formula (2B):

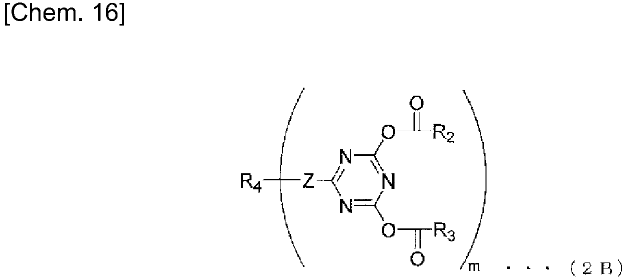

R₂ to R₄ and Z in Formula (2B) have the same meanings as R₂ to R₄ and Z in Formula (2), respectively. When m is an integer of 2 or greater, a plurality of R₂ may be mutually the same or different. Furthermore, a plurality of R₃ may be mutually the same or different.

m in Formula (2B) is an integer of 2 or greater. The upper limit value of m is not particularly limited and can be, for example, 6 or less.

Among these, as preferable examples of the compound represented by Formula (2B), preferable is a compound in which m is an integer from 2 to 4, R₂ and R₃ are each an alkyl group or an aryl group, R₄ is an aliphatic group or an aromatic group, and Z is an oxygen atom or a sulfur atom; more preferable is a compound in which m is an integer from 2 to 3, R₂ and R₃ are each an alkyl group having 3 or less carbons such as a methyl group, an ethyl group, or a propyl group, or a phenyl group, R₄ is an aliphatic group or an aromatic group, and Z is an oxygen atom; still more preferable is a compound in which m is an integer from 2 to 3, R₂ and R₃ are phenyl groups, R₄ is an aromatic group, and Z is an oxygen atom; particularly preferable is a compound in which m is 2, R₂ and R₃ are phenyl groups, R₄ is an aromatic group, and Z is an oxygen atom.

Specific examples of the compound represented by Formula (2B) include the following compounds.

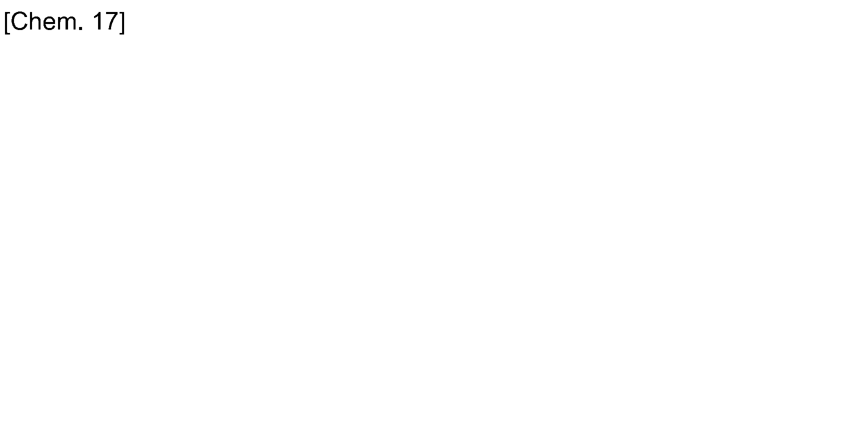

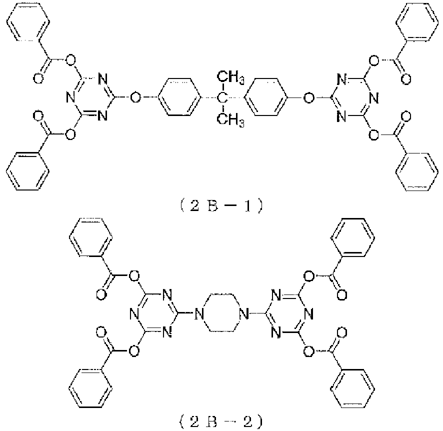

### 1-2. High Molecular Weight Compound

The high molecular weight compound may be a polymer including a constituent unit having a structure in which one acyloxy group is directly bonded to a triazine ring (High Molecular Weight Compound A), or may be a polymer including a constituent unit having a structure in which two acyloxy groups are bonded to a triazine ring (High Molecular Weight Compound B). For example, in the case of a curable composition including a multifunctional epoxy compound and a curing agent, the physical properties of the cured product depend not only on the multifunctional epoxy compound as the main agent but also on the curing agent. Therefore, when the above polymer is used as the curing agent, a cured product reflecting the physical properties of the above polymer can be obtained. For example, using a polymer having a rigid structure as the curing agent can increase the glass transition temperature and mechanical strength of a cured product. Furthermore, using a polymer containing a fluorine atom as the curing agent can lower the dielectric constant of the cured product. Using a polymer containing a sulfur atom, a chlorine atom, or a bromine atom as the curing agent can enhance the flame retardancy of the cured product.

### 1-2-1. High Molecular Weight Compound A

The active ester compound may be a polymer having a structure represented by Formula (3) or (4):

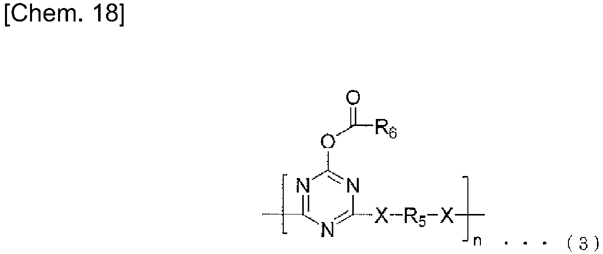

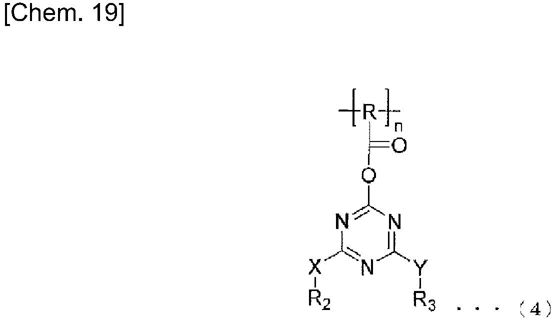

R₂, R₃, and R₆ in Formulae (3) and (4) each have the same meaning as R₂ or R₃ in Formula (2).
R in Formula (4) is a group constituting a repeating unit of a polymer, and is an aliphatic group or an aromatic group. The aliphatic group or aromatic group described above may contain another element in addition to C and H, such as N, O, F, Cl, Br, S, P, or Si.
That is, R is derived from a polymer having a carboxy group in a repeating unit. The number of (COO-) contained in the repeating unit is not limited. Examples of such a polymer include poly(meth)acrylic acid and polyamic acid.
X and Y in Formulae (3) and (4) have the same meanings as X and Y in Formula (2).

In Formula (3), R₅ is a divalent organic group. The divalent organic group has the same meaning as R₄ in Formula (2) where m is 2.

In Formulae (3) and (4), n is an integer of 2 or greater. n is preferably from 2 to 10000, and more preferably an integer from 10 to 100.

Specific examples of the polymer having a structure represented by Formula (3) include the following polymer.

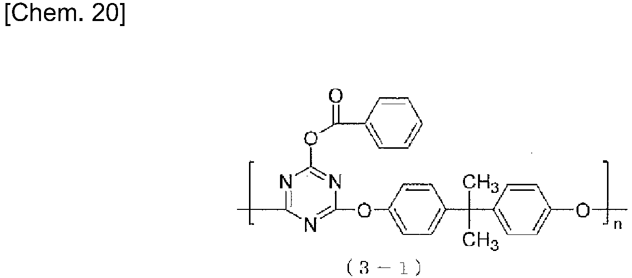

Specific examples of the polymer having a structure represented by Formula (4) include the following polymer.

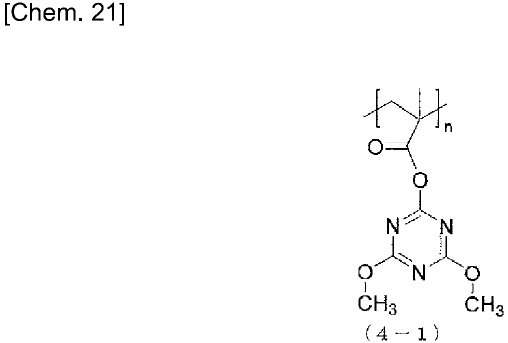

### 1-2-2. High Molecular Weight Compound B

The active ester compound may be a polymer having a structure represented by Formula (5) or (6).

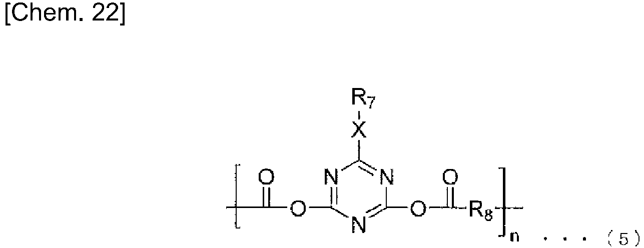

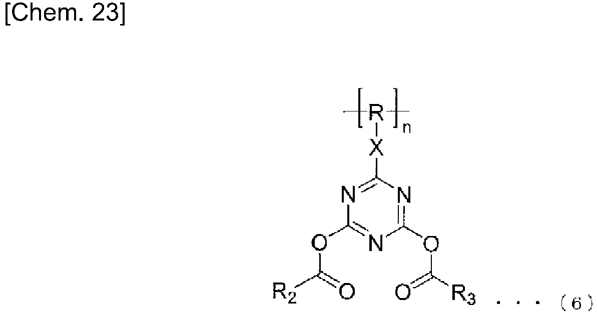

R₇ in Formula (5) has the same meaning as R₂ or R₃ in Formula (2).
R₂ and R₃ in Formula (6) have the same meanings as R₂ and R₃ in Formula (2), respectively.
X in Formulae (5) and (6) has the same meaning as X in Formula (2).
In Formula (5), R₈ is a divalent organic group. The divalent organic group has the same meaning as R₄ in Formula (2) where m is 2.
R in Formula (6) is a group constituting a repeating unit of a polymer, and is an aliphatic group or an aromatic group. The aliphatic group or aromatic group described above may contain another element in addition to C and H, such as N, O, F, Cl, Br, S, P, or Si.
That is, -[R(X)]n- is derived from a polymer having active hydrogen, such as a hydroxy group or an amino group, in a repeating unit. The number of active hydrogens contained in the repeating unit is not limited. Examples of such a polymer include polyphenols (including phenol novolac and cresol novolac) and polyamines. Furthermore, -[R(X)]n- may be a polymer derived from polymerization of a vinyl group.
n in Formulae (5) and (6) is an integer of 2 or greater. n is preferably an integer from 2 to 10000, and more preferably an integer from 10 to 100.

Specific examples of the polymer having a structure represented by Formula (5) include the following polymer.

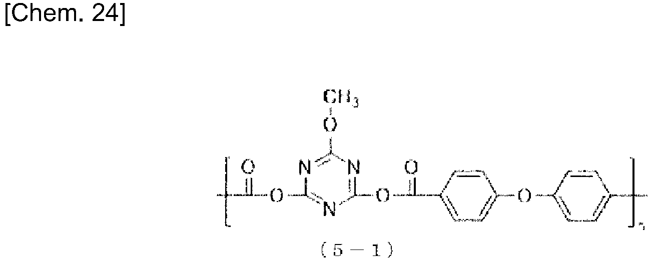

Specific examples of the polymer having a structure represented by Formula (6) include the following polymer.

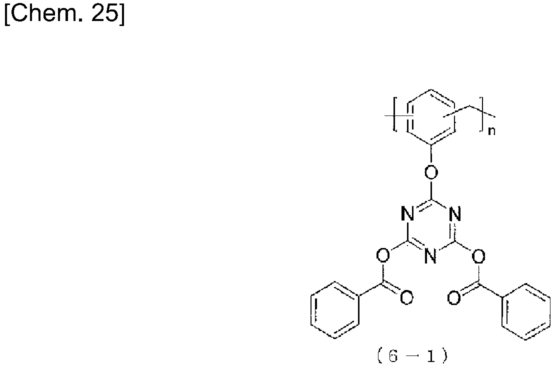

### 1-3. Synthesis Method

The active ester compound can be synthesized, for example, by reacting a chlorotriazine compound with a carboxylic acid compound in an organic solvent in the presence of a tertiary amine compound.

Reaction Scheme (A) can be utilized for the synthesis of a compound having a structure in which one acyloxy group is bonded to a triazine ring (for example, a compound represented by Formula (2A), and a polymer having a structure represented by Formula (3) or (4)).
Reaction Scheme (B) can be utilized for the synthesis of a compound having a structure in which two or more acyloxy groups are bonded to a triazine ring (for example, a compound represented by Formula (2B), and a polymer having a structure represented by Formula (5) or (6)).
Each of the cases will be described below.

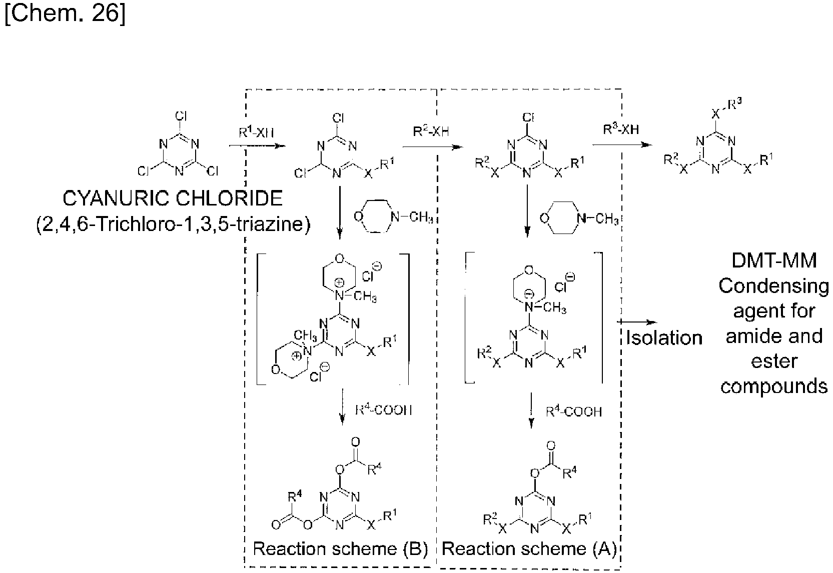

### (1) Synthesis of Compound Represented by Formula (2A) and Polymer Having Structure Represented by Formula (3) or (4)

First, a chlorotriazine compound is prepared (see raw materials in Reaction Scheme (A)).

The chlorotriazine compound is obtained by reacting a part of three chlorine atoms of cyanuric chloride (2,4,6-trichloro-1,3,5-triazine) with a compound having a functional group containing active hydrogen (for example, amine, alcohol, thiol, or the like; this compound is indicated by R-XH in the above reaction scheme) to substitute the part of the three chlorine atoms with the functional group. In Reaction Scheme (A), a compound in which two of three chlorine atoms are substituted with functional groups is used.

Examples of the chlorotriazine compound in which two of three chlorine atoms are substituted with functional groups include 2-chloro-4,6-dimethoxy-1,3,5-triazine, 2-chloro-4,6-diethoxy-1,3,5-triazine, 2-chloro-4-ethoxy-6-methoxy-1,3,5-triazine, and 2-chloro-4,6-diphenoxy-1,3,5-triazine.

Subsequently, the remaining chlorine atom of the above chlorotriazine compound is reacted with a carboxylic acid compound in the presence of a tertiary amine compound, whereby the remaining chlorine atom is substituted with an acyloxy group (see Reaction Scheme (A)).

Specifically, when N-methylmorpholine is used as the tertiary amine compound, the chlorotriazine compound and N-methylmorpholine easily react at 0°C to form an intermediate, 4-(4,6-dimethoxy-1,3,5-triazin-2-yl)-4-methylmorpholinium chloride (DMT-MM), which is then reacted with a carboxylic acid compound. Thereby, an acyloxy group is introduced.

Note that in Reaction Scheme (A), for convenience, the carboxylic acid compound is represented by R⁴-COOH, and only one chlorotriazine compound is illustrated. In the case of obtaining the compound represented by Formula (2A), a polyvalent carboxylic acid may be reacted with a chlorotriazine compound in the same molar equivalent as the molar equivalent of the carboxy groups of the polyvalent carboxylic acid.

As the polyvalent carboxylic acid, a polyvalent carboxylic acid having a number of carboxy groups corresponding to m in Formula (2A) can be used. For example, when m is 2, a dicarboxylic acid can be used, and when m is 3, a tricarboxylic acid can be used.
Examples of the dicarboxylic acid include aromatic dicarboxylic acids such as phthalic acid, isophthalic acid, terephthalic acid, biphenyldicarboxylic acid, naphthalenedicarboxylic acid, oxydibenzoic acid, thiodibenzoic acid, dithiodibenzoic acid, carbonyldibenzoic acid, sulfonyldibenzoic acid, methylenedibenzoic acid, isopropylidenedibenzoic acid, and hexafluoroisopropylidenedibenzoic acid; and aliphatic dicarboxylic acids such as malonic acid, succinic acid, glutaric acid, adipic acid, pimelic acid, suberic acid, azelaic acid, sebacic acid, undecanedicarboxylic acid, and dodecanedicarboxylic acid.
Examples of the tricarboxylic acid include trimesic acid and tricarballylic acid.

Examples of the tertiary amine compound include N-methylmorpholine and pyridine. These tertiary amine compounds not only react well with the chlorotriazine compound, but also can activate the carboxylic acid compound. In addition to these tertiary amine compounds, a tertiary amine compound for activating the carboxylic acid compound may be further used in combination. Examples of such a tertiary amine compound include triethylamine.

Examples of the organic solvent include ketone-based solvents such as acetone, methyl ethyl ketone, and methyl isobutyl ketone; aprotic polar solvents such as N-methyl-2-pyrrolidone, N, N-dimethylacetamide, N-methylcaprolactam, N,N-dimethylimidazolidone, tetramethylurea, pyridine, and γ-butyrolactone; nonpolar solvents such as toluene, hexane, and heptane; ether-based solvents such as tetrahydrofuran, diglyme, dioxane, and trioxane; and mixed solvents thereof.

The blending amount of the tertiary amine compound is preferably from 1.0 to 1.2 molar equivalents relative to the carboxy group of the carboxylic acid compound. Note that the blending amount of the tertiary amine compound is an amount including both the tertiary amine compound for reacting with the chlorotriazine compound and the tertiary amine compound for activating the carboxylic acid described above.
The blending amount of the carboxylic acid compound is preferably the same molar equivalent as the chloro group of the chlorotriazine compound.
The temperature at which the chlorotriazine compound is reacted with the carboxylic acid compound is preferably, for example, from -5 to 25°C.

In the case of synthesizing a polymer having a structure represented by Formula (3), for example, a chlorotriazine compound made to have a high molecular weight by polycondensation or the like may be used as the raw material.
In the case of synthesizing a polymer having a structure represented by Formula (4), chlorotriazine may be introduced into a polymer having a carbonyl group in its side chain, such as polyacrylic acid, polymethacrylic acid, or polyamic acid.

### (2) Synthesis of Compound Represented by Formula (2B) and Polymer Having Structure Represented by Formula (5) or (6)

First, a chlorotriazine compound in which one of three chlorine atoms of cyanuric chloride is substituted with a functional group is prepared (see raw materials in Reaction Scheme (B)).

Subsequently, the remaining two chlorine atoms of the above chlorotriazine compound are reacted with a carboxylic acid compound in the presence of a tertiary amine compound, whereby two chlorine atoms are substituted with acyloxy groups (see Reaction Scheme (B)).

As the carboxylic acid compound, a monocarboxylic acid can be used. The monocarboxylic acid may be aliphatic or aromatic, and may be an unsaturated compound. Furthermore, another atom may be bonded in or on the ring. Examples of the monocarboxylic acid include aliphatic saturated monocarboxylic acids (for example, formic acid, acetic acid, propionic acid, butyric acid, isobutyric acid, valeric acid, isovaleric acid, pivalic acid, lauric acid, myristic acid, palmitic acid, stearic acid), aliphatic unsaturated monocarboxylic or dicarboxylic acids (for example, acrylic acid, propiolic acid, methacrylic acid, crotonic acid, isocrotonic acid, senecioic acid, tiglic acid, oleic acid, maleic acid, fumaric acid, citraconic acid, mesaconic acid), saturated or unsaturated carbocyclic carboxylic acids (for example, camphoric acid, chrysanthemic acid), heterocyclic carboxylic acids (for example, furancarboxylic acid, thiophenecarboxylic acid, pyrrolecarboxylic acid, pyrazinecarboxylic acid, nicotinic acid, isonicotinic acid, picolinic acid), aromatic carbocyclic carboxylic acids (for example, benzoic acid, naphthalenecarboxylic acid, toluene acid), arylaliphatic saturated carboxylic acids, particularly arylpropionic acids (for example, 2-phenylpropionic acid, 2-[4-(2-butyl)phenyl]propionic acid, 2-(3-benzoylphenyl) propionic acid, 2-(6-methoxy-2-naphthyl)propionic acid) or unsaturated acids (for example, 2-phenylpropenoic acid, cinnamic acid), aliphatic or aromatic halogenated carboxylic acids (for example, monofluoroacetic acid, difluoroacetic acid, monochloroacetic acid, dichloroacetic acid, trichloroacetic acid, monochloropropionic acid, α-bromopropionic acid, α-bromobutyric acid, trifluoroacetic acid, o-monofluorobenzoic acid, m-monofluorobenzoic acid, p-monofluorobenzoic acid, 2,3-difluorobenzoic acid, 2,4-difluorobenzoic acid, 2,5-difluorobenzoic acid, 3,4-difluorobenzoic acid, 2,3,6-trifluorobenzoic acid, 2,4,5-trifluorobenzoic acid, 2,3,4,5-tetrafluorobenzoic acid, pentafluorobenzoic acid, α,α,α-trifluoro-o-toluic acid, α,α,α-trifluoro-m-toluic acid, α,α,α-trifluoro-p-toluic acid, o-monochlorobenzoic acid, m-monochlorobenzoic acid, p-monochlorobenzoic acid, 2,3-dichlorobenzoic acid, 2,4-dichlorobenzoic acid, 2,5-dichlorobenzoic acid, 2,6-dichlorobenzoic acid, 3,4-dichlorobenzoic acid, 3,5-dichlorobenzoic acid, 2,3,5-trichlorobenzoic acid, 2,3,6-trichlorobenzoic acid, 2-chloro-4, 5-fluorobenzoic acid, 3-chloro-2,4,5-trifluorobenzoic acid, o-monobromobenzoic acid, m-monobromobenzoic acid, p-monobromobenzoic acid).

As the tertiary amine compound and the organic solvent, the same compounds and solvents as described above can be used.

Note that in Reaction Scheme (B), for convenience, a chlorotriazine compound having one chlorotriazine ring is illustrated. In the case of synthesizing the compound represented by Formula (2B), a chlorotriazine compound having a plurality of chlorotriazine rings, which is obtained by reacting cyanuric chloride with a multifunctional compound, may be used.

In the case of synthesizing a polymer having a structure represented by Formula (5), a dicarboxylic acid may be used as the carboxylic acid compound. For example, the dichlorotriazine compound and a dicarboxylic acid may be reacted and polycondensed in the presence of a tertiary amine.
In the case of synthesizing a polymer having a structure represented by Formula (6), a chlorotriazine compound having a polymerizable reactive group such as a vinyl group introduced as a functional group may be used, or a chlorotriazine compound reacted with a polymer having active hydrogen such as a hydroxy group or an amino group in a repeating unit may be used. For example, a chlorotriazine structure-containing polymer in which one of the chlorine atoms of cyanuric chloride is added through substitution to hydroxy groups of phenol novolac or the like can be prepared, and the remaining chlorine atoms of chlorotriazine can be substituted with acyloxy groups.

The structure of the active ester compound can be identified by ¹H NMR measurement, ¹³C NMR measurement, FT-IR measurement, and MS measurement.

### ¹H NMR Measurement

The ¹H NMR measurement can be performed using an NMR measurement apparatus (for example, JNM-ECZ600R/S1 available from JEOL Ltd.). Specifically, a sample for measurement is adjusted by adding 20 mg of white powder and 0.5 mL of deuterated chloroform to a vial, and the measurement is performed under the following conditions: magnetic field strength: 600 MHz, number of scans: 8. The obtained results are normalized such that the resonance peak of tetramethylsilane in the deuterated solvent appears at 0.00 ppm.

### ¹³C NMR Measurement

The ¹³C NMR measurement is performed by adjusting a sample in the same manner as in the ¹H NMR measurement, and using the same apparatus under the following conditions: magnetic field strength: 600 MHz, number of scans: 128. The obtained results are normalized such that the resonance peak of chloroform appears at 77.0 ppm. In Formulae (1) to (6) above, in the case of an active ester compound in which the X and Y atoms bonded to the triazine ring are oxygen atoms and a hydrocarbon group is bonded to the oxygen atoms, characteristic peaks derived from the triazine ring are observed at 171 and 174 ppm. Furthermore, one peak derived from the acyloxy group bonded to the triazine ring is observed at 160 to 170 ppm.

### FT-IR Measurement

The Fourier transform infrared spectroscopy (FT-IR) measurement can be performed by a KBr method using an FT-IR apparatus (for example, FT/IR-4100 available from JASCO Corporation). Specifically, 1.0 mg of white powder and 100 mg of KBr powder are homogeneously mixed, and then placed into a tableting machine and tableted under pressure to prepare a test sample. Thereafter, the spectrum is measured under the following conditions: number of scans: 25, resolution: 2 cm⁻¹. In Formulae (1) to (6) above, in the case of an active ester compound in which the X and Y atoms bonded to the triazine ring are oxygen atoms and a hydrocarbon group is bonded to the oxygen atoms, stretching vibration of ester C=O is observed near 1750 cm⁻¹. In addition, a strong peak derived from triazine-ring skeletal vibration is observed near 1360 cm⁻¹.

### MS Measurement

Mass spectrometry (MS) is performed by atmospheric pressure chemical ionization (APCI) and field desorption-mass spectroscopy (FD-MS).
For the APCI, white powder is dissolved in a solvent (acetone), and a sample solution is adjusted so as to have a sample concentration of 10 mg/mL. Thereafter, the sample solution is subjected to filtering, and the mass spectrometry can be performed using a mass spectrometer (for example, CMS expression available from Advion, Inc.) under the following conditions: mobile phase: acetone, nebulizer gas: nitrogen, "low temperature low fragmentation". For the FD-MS, white powder is dissolved in tetrahydrofuran, and a sample solution is adjusted so as to have a sample concentration of 2 mg/mL. Thereafter, the sample solution is subjected to filtering, and the mass spectrometry can be performed using a mass spectrometer (for example, GC-TOFMS JMS-T100GCV available from JEOL Ltd.) under the following conditions: measurement mode: eiFI FD+, cathode (emitter-applied) voltage: - 10 kV, detector voltage: 2.4 kV, emitter-current: from 0 mA to 40 mA (increasing rate of 51.2 mA/min). A multifunctional active ester compound has a high molecular weight, and thus a peak of the corresponding molecular weight may not be obtained in MS measurement, but the corresponding triazinolate is detected as a fragment peak. For example, in Formulae (1) to (6) above, in the case of an active ester compound in which the X and Y atoms bonded to the triazine ring are oxygen atoms and methyl groups are bonded to the oxygen atoms (an active ester compound in which methoxy groups are bonded to the triazine ring), a peak derived from m/z = 158 [2,4-dimethoxy-6-hydroxy-1,3,5-triazine+1]⁺ is detected.

### 2. Method of Reacting Epoxy Compound

The active ester compound according to the present embodiment can undergo an addition reaction with an epoxy group of an epoxy compound even at a low temperature, and can impart functionality to the epoxy compound by introducing a functional group into a substituent other than the acyloxy group of the triazine ring. When the epoxy compound and the active ester compound are each a multifunctional compound, they can be preferably used as a curing agent for the epoxy compound.

That is, a curable composition including an epoxy compound and a curing agent including the above active ester compound can be provided. The resulting cured product does not contain a hydroxy group formed by the ring-opening of the epoxy group, and therefore, increases in hygroscopicity and dielectric constant can be suppressed.

### 2-1. Curable Composition

The curable composition includes an epoxy compound and the active ester compound. The curable composition has a property of being cured (solidified) under specific conditions, and is usually in the form of a liquid or a varnish.

### Epoxy Compound

The epoxy compound is not particularly limited as long as it is a compound having two or more epoxy groups per molecule, and may be an alicyclic epoxy compound, a glycidyl-type epoxy compound including glycidyl ether, glycidyl amine, and glycidyl ester, or an internal epoxide. The epoxy compound may be a low molecular weight compound or a high molecular weight compound. When the epoxy compound is a high molecular weight compound, the epoxy compound may have an epoxy group at the terminal or one or more epoxy groups in a repeating unit constituting the high molecular weight compound. The above active ester compound exhibits high reactivity particularly with an alicyclic epoxy compound.

In general, an alicyclic epoxy compound has lower reactivity than a glycidyl-type epoxy compound. Therefore, in order to cure an alicyclic epoxy compound, it is usually necessary to increase the reaction temperature as compared with the case of a glycidyl-type epoxy compound. In contrast, the above active ester compound has high reactivity, and therefore can react with an alicyclic epoxy compound at a low temperature as in the case of a glycidyl-type epoxy compound.

### Active Ester Compound

Among the above active ester compounds, from the viewpoint of increasing the reactivity with the epoxy compound, an active ester compound in which X and Y in Formulae (1) to (5) above are -O- or -S- is preferable. In addition, from the viewpoint of combining with an alicyclic epoxy compound, at least one of R₂ or R₃ is preferably an alkyl group or an aryl group, at least one of R₂ or R₃ is more preferably an alkyl group having 3 or less carbons such as a methyl group, an ethyl group, or a propyl group, or a phenyl group, and at least one of R₂ or R₃ is still more preferably a methyl group.

Thus, the reactivity of the active ester compound depends to some extent on the structure of the active ester compound, particularly the structure of R₁, X, and Y.
That is, the reactivity of the above active ester compound with the epoxy compound largely depends on the electron-withdrawing property of R₁ and the stability of the 1,3,5-triazin-2-olate to be eliminated. 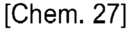 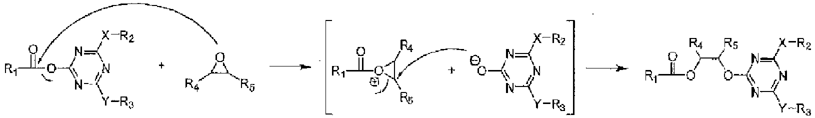 For example, in the above reaction scheme, as the electron donating properties of X and Y of the active ester compound are lower, the electron density of the triazine ring is less likely to increase; therefore, the 1,3,5-triazin-2-olate to be produced is more likely to be stabilized, and the reactivity is more likely to be higher. In addition, as the electron-donating properties of R₂ and R₃ of the active ester compound are lower, the electron-donating properties of X and Y are also lower; therefore, the reactivity is likely to be still higher. Specifically, when X and Y are -O- or -S-, the electron donating properties of X and Y are lower than that of -NR-; therefore, the reactivity between the active ester compound and the epoxy compound is likely to be higher. That is, the reactivity of the active ester compound depends on X and Y.

Therefore, even if R₂ and R₃ are different, the active ester compounds having R₁, X and Y in common to some extent exhibit equivalent reactivity. In addition, if a low molecular weight compound exhibits reactivity equal to or higher than a predetermined level, a corresponding high molecular weight substance (high molecular weight compound) also exhibits nearly similar reactivity. That is, even if R₂ or R₃ of the active ester compound has a complicated structure or a high molecular weight structure, as long as R₁, X, and Y (particularly, X and Y) of the active ester compound are equivalent, similar reactivity (reaction temperature) is exhibited.

### Additive

The reaction between the epoxy compound and the active ester compound may be performed in the presence of an additive from the viewpoint of further increasing the reaction efficiency. The additive is not particularly limited as long as it promotes the reaction between the epoxy compound and the active ester compound, but is usually a curing accelerator such as a nucleophilic catalyst. Examples of the curing accelerator include a phosphorus-based curing accelerator, an amine-based curing accelerator, and an imidazole-based curing accelerator.
Among these, an amine-based curing accelerator is preferable from the viewpoint of further increasing the reactivity between the active ester compound and the epoxy compound. Among the amine-based curing accelerators, those having a heterocyclic structure such as pyridine and pyrimidine are preferable, those having a pyridine structure are more preferable, and 4-dimethylaminopyridine is still more preferable.

### Reactivity with Alicyclic Epoxy Compound

As described above, the active ester compound has high reactivity also with an alicyclic epoxy compound. The reaction between the alicyclic epoxy compound and the active ester compound is more likely to be completed at a lower temperature than that for the reaction between other epoxy compounds and the active ester compound, and the reaction time can be further shortened. The reason for this is not clear but is presumed as follows.

For example, an explanation will be given using an example in which the epoxy compound and the active ester compound are reacted in the presence of an additive. When a glycidyl-based epoxy compound is used, the additive reacts not only with the active ester compound but also with the glycidyl-based epoxy compound. In contrast, when an alicyclic epoxy compound is used, the additive reacts with the active ester compound but does not react with the alicyclic epoxy compound. Thus, the reaction mechanisms are different between the case of using an alicyclic epoxy compound and the case of using a glycidyl-based epoxy compound, and the reaction scheme is simpler in the case of using the alicyclic epoxy compound than in the case of using the glycidyl-based epoxy compound. Therefore, the reaction between an alicyclic epoxy compound and the active ester compound is more likely to be completed at a lower temperature, and the reaction time can be further shortened.

### 3. Method of Producing Curable Composition

The curable composition can be produced by any method. For example, the curable composition can be produced by a method in which components such as an epoxy compound, the active ester compound, and, if necessary, an additive are dissolved in a solvent and mixed, and then the solvent is removed.

Here, the curable composition obtained by the above production method is in the form of a varnish, and is preferably a homogeneous varnish even after the solvent is distilled off from the viewpoint of workability and storage stability. Therefore, in order to obtain a homogeneous varnish, it is desired to further increase the compatibility between the active ester compound and the epoxy compound.

In order to increase compatibility between the active ester compound and the epoxy compound, when the curable composition is produced, it is preferable to heat the active ester compound and the epoxy compound to a temperature equal to or higher than the reaction initiation temperature thereby to partially react these compounds. This makes it possible to further enhance the compatibility between the active ester compound and the epoxy compound, and therefore, for example, it is possible to further suppress the precipitation of the solid content when the solvent is distilled off. According to the above production method, a homogeneous curable composition having higher compatibility can be obtained. Therefore, even when the curable composition is stored for a long period of time, separation of the components of the curable composition can be further suppressed.

The heating temperature for partially reacting the active ester compound with the epoxy compound may be equal to or higher than the temperature at which the epoxy compound reacts with the active ester compound. The heating temperature is preferably, for example, Tp - 30°C or higher and Tp + 30°C or lower, where Tp is defined as a reaction temperature measured by DSC described later. When the temperature is Tp - 30°C or higher, the compatibility between the epoxy compound and the active ester compound can be further enhanced. When the temperature is Tp + 30°C or lower, gelation of the composition due to excessive curing of the epoxy compound can be suppressed. When the heating temperature is high, gelation tends to occur more easily. Therefore, when the heating temperature is high, shortening the heating time can suppress gelation. The heating temperature can be, for example, 70°C or higher and 190°C or lower.

The reaction temperature Tp can be measured by the following method. The epoxy compound and the active ester compound to be used are mixed in a mortar until they become homogeneous. The obtained composition is subjected to DSC measurement, the temperature is raised from 0°C to 200°C at 10°C/min, and the peak top temperature of the exothermic peak observed first is read and taken as the reaction temperature Tp.

As described above, the active ester compound has a low reaction temperature with the epoxy compound, and therefore can be reacted with the epoxy compound even at a low temperature at which the solvent does not volatilize. Therefore, the above method is particularly effective.

### 4. Curable Composition Applications

The cured product of the above curable composition can have excellent dielectric properties because active hydrogen is not generated. Accordingly, the curable composition can be used for electronic material applications such as semiconductor package substrates, printed wiring boards, build-up films, and semiconductor sealing materials. In addition, the curable composition can be applied to applications such as adhesives and paints.

### 5. Others

In the present embodiment, the structure of the active ester compound preferable for increasing the reactivity with the epoxy compound, the type of epoxy compound to be combined, and the additive have been described, but these descriptions are also applicable to the first embodiment.

In addition, in the present embodiment, as the method of producing the curable composition, a method of heating the active ester compound and the epoxy compound to a temperature equal to or higher than the reaction temperature thereby to partially react these compounds has been described, but the above method may be applied to the method for producing the composition of the first embodiment.

### EXAMPLES

Hereinafter, the present invention will be described further in detail with reference to examples, but the present invention is not limited to these examples.

### 1. Preparation of Monofunctional Active Ester Compound

### Synthesis of Compound A

To a 50 mL three-neck flask equipped with a 50 mL dropping funnel, a magnetic stirrer, and a thermometer, 10 mL of tetrahydrofuran (THF) (dehydrated) and 1.76 g (0.010 mol, 144 parts by mass) of 2-chloro-4,6-dimethoxy-1,3,5-triazine were added and dissolved at a temperature of 25°C, followed by cooling to 0°C to obtain a first solution. 1.05 g (0.010 mol, 86 parts by mass) of N-methylmorpholine was added dropwise to the first solution and mixed therewith in such a manner that the temperature of the first solution did not exceed 5°C. To another beaker, 10 mL of THF (dehydrated) and 1.22 g (0.010 mol, 100 parts by mass) of benzoic acid were added and dissolved to obtain a second solution. The second solution was added dropwise to the first solution and mixed therewith in such a manner that the temperature of the first solution did not exceed 5°C, thereby obtaining a reaction solution. The reaction solution was stirred for 1 hour while maintaining the temperature at 0°C, and then stirring was continued at a temperature of 25°C for 1 hour. After the stirring, the reaction solution was filtered to remove the precipitated salt. The solvent was removed from the filtrate using an evaporator, and 20 mL of ethyl acetate was added to the residue to dissolve it. The solution was transferred to a separatory funnel and washed once with 20 mL of water, three times with 20 mL of 0.5 M aqueous sodium hydrogen carbonate solution, and once with 20 mL of saturated brine. The solution after the washing was transferred to an Erlenmeyer flask. An appropriate amount of anhydrous magnesium sulfate was added thereto to dehydrate the mixture, followed by filtration. The solvent was removed from the filtrate using an evaporator, and the filtrate was dried under reduced pressure at a temperature of 25°C, thereby obtaining 1.68 g of a white powder (Compound A) in a yield of 64.3%.

### Identification

The white powder was subjected to the following measurements to identify the structure.

### ¹H NMR Measurement, ¹³C NMR Measurement

The ¹H NMR measurement was performed using an NMR measurement apparatus (JNM-ECZ600R/S1 available from JEOL Ltd.) More specifically, a sample for measurement was adjusted by adding 20 mg of the white powder and 0.5 mL of deuterated chloroform to a vial, and the measurement was performed under the following conditions: magnetic field strength: 600 MHz, number of scans: 8. The obtained results were normalized such that the resonance peak of tetramethylsilane in the deuterated solvent appeared at 0.00 ppm.
The ¹³C NMR measurement was performed by adjusting a sample in the same manner as in the ¹H NMR measurement, and using the same apparatus under the following conditions: magnetic field strength: 600 MHz, number of scans: 128. The obtained results were normalized such that the resonance peak of chloroform appeared at 77.0 ppm.
In each measurement, the chemical shift delta δ is expressed in ppm. The coupling constant J is expressed in hertz (Hz). Note that the notations d and t represent d (doublet) and t (triplet), respectively.

### FT-IR Measurement

The Fourier transform infrared spectroscopy (FT-IR) measurement was performed by a KBr method using an FT-IR apparatus (FT/IR-4100 available from JASCO Corporation). More specifically, 1.0 mg of the white powder and 100 mg of KBr powder were homogeneously mixed, and then placed into a tableting machine and tableted under pressure to prepare a test sample. Thereafter, the spectrum was measured under the following conditions: number of scans: 25, resolution: 2 cm⁻¹.

### MS Measurement

Mass spectrometry (MS) was performed by atmospheric pressure chemical ionization (APCI) and field desorption-mass spectroscopy (FD-MS).
For the APCI, the white powder was dissolved in a solvent (acetone), and a sample solution was adjusted so as to have a sample concentration of 10 mg/mL. Thereafter, the sample solution was subjected to filtering, and the mass spectrometry was performed using a mass spectrometer (CMS expression available from Advion, Inc.) under the following conditions: mobile phase: acetone, nebulizer gas: nitrogen, "low temperature low fragmentation". For the FD-MS, the white powder was dissolved in tetrahydrofuran, and a sample solution was adjusted so as to have a sample concentration of 2 mg/mL. Thereafter, the sample solution was subjected to filtering, and the mass spectrometry was performed using a mass spectrometer (GC-TOFMS JMS-T100GCV available from JEOL Ltd.) under the following conditions: measurement mode: eiFI FD+, cathode (emitter-applied) voltage: -10 kV, detector voltage: 2.4 kV, emitter-current: from 0 mA to 40 mA (increasing rate of 51.2 mA/min).

### Results

The results of ¹H NMR measurement, ¹³C NMR measurement, FT-IR measurement, and MS measurement of the white powder are as follows:
¹H NMR (600 MHz, CDCl₃, TMS): δ = 8.15 (d, J = 7.5 Hz, 2H), 7.81 (t, J = 7.5 Hz, 1H), 7.68 (t, J = 7.5 Hz, 2H), 4.06 (s, 6H);
¹³C NMR (100 MHz, CDCl₃): δ = 174.3 (triazine), 172.7 (triazine), 171.0 (triazine), 162.6 (C=O), 134.6 (phenyl group), 130.7 (phenyl group), 128.9 (phenyl group), 128.8 (phenyl group), 56.1 (CH₃);
FT-IR (KBr, cm⁻¹): 3083 (Ar-H stretching vibration), 2952 (CH₃ stretching vibration), 1750 (C=O stretching vibration), 1574 (benzene ring skeletal vibration), 1558 (triazine ring skeletal vibration), 1449 (phenyl group skeletal vibration), 1228 (phenyl group skeletal vibration); and
MS (APCI): m/z 262.08 [M+H⁺]⁺.
From these analysis results, it was confirmed that the white powder (Compound A) was 2,4-dimethoxy-6-benzoyloxy-1,3,5-triazine.

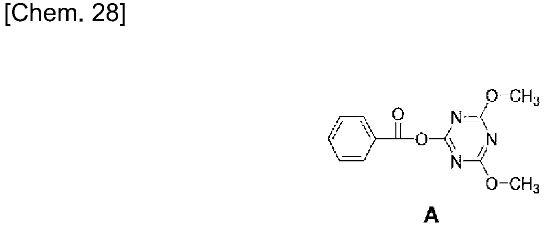

### Synthesis of Compounds B to E

Compounds B to E were synthesized in the same manner as in the case of Compound A except that the types of raw materials were changed.

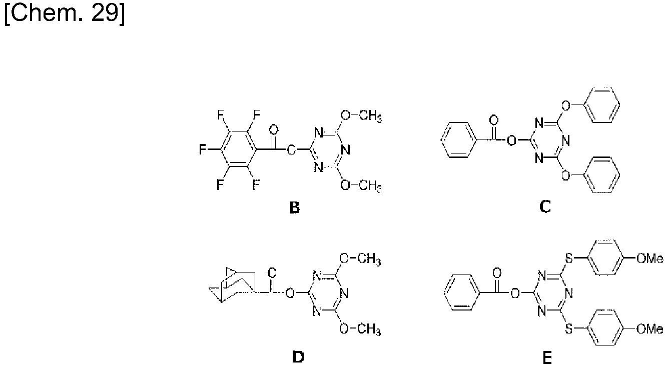

### Nitrophenyl Acetate

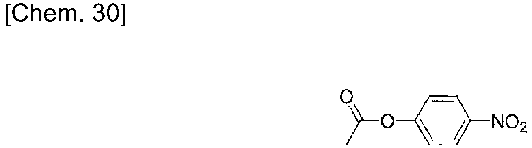

### 2. Evaluation of Monofunctional Active Ester Compound

### 2-1. Reactivity

### Example 1

Compound A was added to Epoxy Compound I (jER828 available from Mitsubishi Chemical Corporation, bisphenol A-type epoxy resin, see the following formula) such that the molar equivalent of the ester group of Compound A was equal to the molar equivalent of the epoxy group of Epoxy Compound I. Furthermore, as an additive, 2 parts by mass of dimethylaminopyridine (DMAP) relative to the epoxy compound was added thereto, and the mixture was mixed in a mortar until it became homogeneous, to obtain a composition. 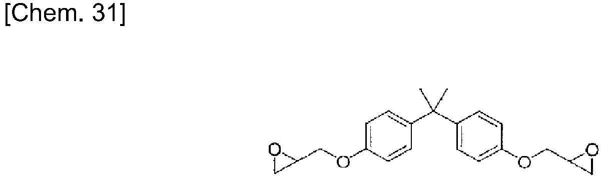 The reaction temperature with the epoxy compound was determined by DSC (differential scanning calorimetry) measurement. Specifically, 10 mg of the obtained composition was placed in an aluminum pan, and was measured using a differential scanning calorimeter (DSC 3+ available from METTLER TOLEDO) under a nitrogen gas stream while temperature was raised from 0°C to 200°C at 10°C/min. The peak top temperature of the exothermic peak observed first was read and taken as the reaction temperature. The reaction temperature was 131°C.

### Examples 2 to 5

The reaction temperature was measured in the same manner as in Example 1 except that the compounds listed in Table 1 were used instead of Compound A.

### Comparative Example 1

The reaction temperature was measured in the same manner as in Example 1 except that nitrophenyl acetate, which is a general active ester compound, was used instead of Compound A. As a result, the reaction temperature was 184°C.

### Evaluation Results

The evaluation results are shown in Table 1.

**[Table 1]**

| | Reactant type | Number of functional groups of reactant | Epoxy compound | Additive | Molar ratio of ester group/epoxy group in composition | Ester compound [mg] | Epoxy compound + additive [mg] | Additive [parts by mass/100 parts by mass of epoxy compound] | Peak temperature (°C) |
|---|---|---|---|---|---|---|---|---|---|
| Example 1 | A | Monofunctional | | | 1.0 | 145.7 | 94.8 | 2.0 | 131 |
| Example 2 | B | Monofunctional | | | 1.0 | 195.1 | 95.3 | 2.0 | 145 |
| Example 3 | C | Monofunctional | Epoxy Compound I | | 1.0 | 70.6 | 31.2 | 2.0 | 148 |
| Example 4 | D | Monofunctional | | DMAP | 1.0 | 181.4 | 96.6 | 2.0 | 129 |
| Example 5 | E | Monofunctional | | | 1.0 | 366.3 | 94.8 | 2.0 | 158 |
| Comparative Example 1 | Nitrophenyl acetate | Monofunctional | | | 1.0 | 135.8 | 127.4 | 2.0 | 184 |

### Discussion

As shown in Table 1, it can be seen that Compounds A to E (Examples 1 to 5) have lower peak temperatures than nitrophenyl acetate (Comparative Example 1) and react with the epoxy compound at a lower temperature.

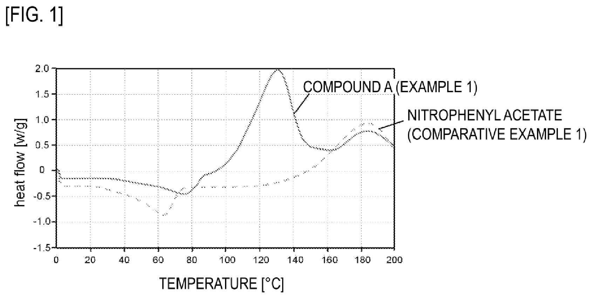

FIG. 1 is a graph showing the results of DSC measurement of the monofunctional active ester compound. As shown in FIG. 1, it can be seen that Compound A (Example 1) reacts with the epoxy compound at a lower temperature than that for nitrophenyl acetate (Comparative Example 1).

### 2-2. Confirmation of Reaction

### Experimental Method

Compound A was added to glycidyl phenyl ether (GPE) as an epoxy compound such that the molar equivalent of the ester group of Compound A was equal to the molar equivalent of the epoxy group of the GPE. Furthermore, DMAP was added in an amount of 1 mass% relative to the entire composition, and the mixture was mixed in a mortar until it became homogeneous to obtain a composition. Subsequently, the above composition was added to a flask, which was then heated to 130°C using an oil bath while stirring. The heated products were aliquoted after 0.5 hours, 1 hour, 2 hours, 3 hours, and 4 hours of heating.

### ¹H NMR Measurement

In order to confirm the degree of progress of the reaction between the epoxy compound and Compound A, ¹H NMR measurement was performed on each of the aliquoted heated products. The ¹H NMR measurement was performed using an NMR measurement apparatus (JMM-ECZ600R/S1 available from JEOL Ltd.). More specifically, a sample for measurement was adjusted by adding 20 mg of the obtained heated product and 0.5 mL of a deuterated solvent (chloroform-d) to a vial, and then measured under the following conditions: magnetic field strength: 600 MHz, number of scans: 8. The obtained results were normalized such that the resonance peak of tetramethylsilane appeared at 0 ppm. As a result, as the heating time increased, the peak at 2.7 to 2.9 ppm derived from the epoxy group decreased, and the peak at 5.6 ppm derived from the reaction product increased. In the heated product aliquoted after 3 hours of heating, no peak at 2.7 to 2.9 ppm derived from the epoxy group was observed. These results indicated that the reaction of the ester group of Compound A with the epoxy group of the epoxy compound started immediately at 130°C, proceeded with time, and had almost completed at 3 hours.

### MS Measurement

Each of the aliquoted heated products was subjected to mass spectrometry by atmospheric pressure chemical ionization (APCI). Specifically, the resulting heated product was dissolved in a solvent (acetone), and a sample solution was adjusted so as to have a sample concentration of 10 mg/mL. Thereafter, the sample solution was subjected to filtering, and the mass spectrometry was performed using a mass spectrometer (CMS expression available from Advion, Inc.) under the following conditions: mobile phase: acetone, nebulizer gas: nitrogen, "low temperature low fragmentation". As a result, a peak of m/z = 412 was observed in all the heated products obtained at the respective heating times. This corresponds to [M+1]⁺ of the addition product of Compound A and the epoxy compound.

From the results of NMR measurement and MS measurement, it was confirmed that the epoxy group of the epoxy compound and Compound A underwent an addition reaction.

### 3. Preparation of Multifunctional Active Ester Compound

### Synthesis of Compound F

To a 500 mL three-neck flask equipped with a 200 mL dropping funnel, a calcium chloride tube, a magnetic stirrer, and a thermometer, 200 mL of THF (dehydrated) and 12.6 g (0.072 mol, 249 parts by mass) of 2-chloro-4,6-dimethoxy-1,3,5-triazine were added and dissolved at a temperature of 25°C, followed by cooling to 0°C to obtain a first solution.
To another beaker, 50 mL of THF (dehydrated) and 8.69 g (0.086 mol, 172 parts by mass) of N-methylmorpholine were added and dissolved to obtain a second solution. The second solution was added dropwise to the first solution and mixed therewith such that the temperature of the first solution did not exceed 5°C, thereby obtaining a mixed solution.
Subsequently, to another beaker, 70 mL of THF (dehydrated) and 5.06 g (0.024 mol, 100 parts by mass) of 1,3,5-benzenetricarboxylic acid were added and dissolved to obtain a third solution. The third solution was added dropwise to the mixed solution and mixed therewith such that the temperature of the mixed solution did not exceed 5°C, thereby obtaining a reaction solution. The reaction solution was stirred for 1 hour while maintaining the temperature at 0°C. After the stirring, the reaction solution was filtered to remove the precipitated salt. The filtrate was transferred to a separatory funnel and washed three times with 120 mL of 10% brine adjusted to pH 2 with hydrochloric acid. The filtrate was further washed once with 100 mL of saturated brine, and the washed filtrate was separated into an Erlenmeyer flask. An appropriate amount of magnesium sulfate was added thereto to dehydrate the mixture, followed by filtration. The solvent was removed from the filtrate using an evaporator, followed by drying under reduced pressure in a desiccator at a temperature of 25°C for 21 hours to obtain 8.12 g of a white powder (Compound F) in a yield of 54.1%.

### Identification

The obtained white powder was subjected to the same measurements as above to identify the structure.

### Results

The results of ¹H NMR measurement, ¹³C NMR measurement, FT-IR measurement, and MS measurement of the white powder are as follows:
¹H NMR (600 MHz, DMSO-d6, TMS): δ = 8.67 (s, 3H), 3.91 (s, 6H);
¹³C NMR (150 MHz, DMSO-d6): δ = 173.5 (triazine), 168.5 (triazine), 166.4 (triazine), 157.5 (C=O), 133.8 (phenyl group), 132.4 (phenyl group), 130.9 (phenyl group), 55.7 (CH₃);
FT-IR (KBr, cm⁻¹): 3069 (Ar-H stretching vibration), 2954 (CH₃ stretching vibration), 1722 (C=O stretching vibration), 1600 (benzene ring skeletal vibration), 1540 (triazine ring skeletal vibration), 1241 (phenyl group skeletal vibration), 1099 (phenyl group skeletal vibration); and
MS(FD-MS): m/z 628.14 [M+H⁺]⁺.
From these analysis results, it was confirmed that the white powder (Compound F) was a triazine compound represented by the following formula.

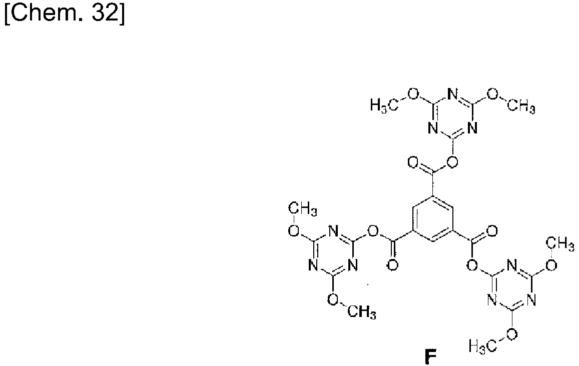

### Synthesis of Compound H

To a 50 mL three-neck flask equipped with a 50 mL dropping funnel, a magnetic stirrer, and a thermometer, 20 mL of THF (dehydrated) and 1.93 g (0.010 mol, 150 parts by mass) of 2-chloro-4,6-di(p-methoxybenzenethio)-1,3,5-triazine, and 1.29 g (0.005 mol, 100 parts by mass) of 4,4'-dicarboxydiphenyl ether were added and dissolved at a temperature of 25°C, followed by cooling to 0°C to obtain a first solution.
To another beaker, 50 mL of THF (dehydrated) and 1.52 g (0.015 mol, 118 parts by mass) of N-methylmorpholine were added and dissolved to obtain a second solution. The second solution was added dropwise to the first solution and mixed therewith such that the temperature of the first solution did not exceed 5°C, thereby obtaining a mixed solution. The reaction solution was stirred for 1 hour while maintaining the temperature at 0°C, and then continued stirring at 25°C for 1 hour. After the stirring, the reaction solution was filtered to remove the precipitated salt. The solvent was removed from the filtrate using an evaporator, and the residue was dissolved in 20 mL of chloroform. The solution was transferred to a separatory funnel and washed twice with 20 mL of water. The obtained organic layer was dried over anhydrous magnesium sulfate, the solid was filtered off, and the solvent was removed using an evaporator to obtain a yellowish white powder (Compound H) in a yield of 84.1%.

### Synthesis of Compounds G to U

Compounds R to U were synthesized in the same manner as in the case of Compound F except that the types and amounts of the raw materials were changed. In addition, Compounds G to Q were synthesized in the same manner as in the case of Compound H except that the types and amounts of the raw materials were changed.

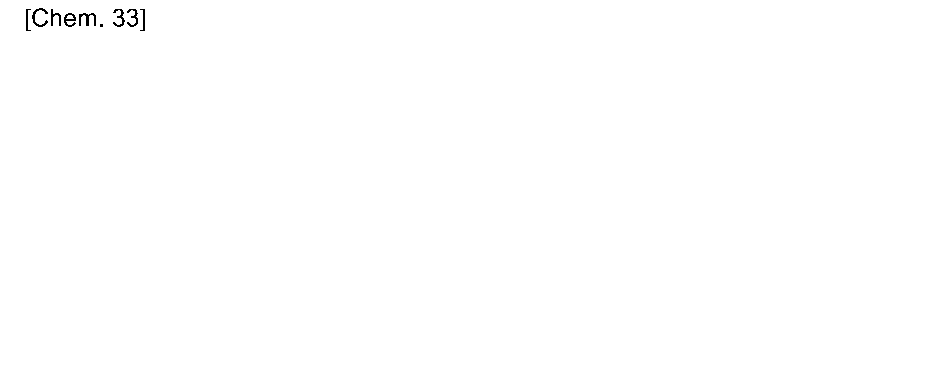

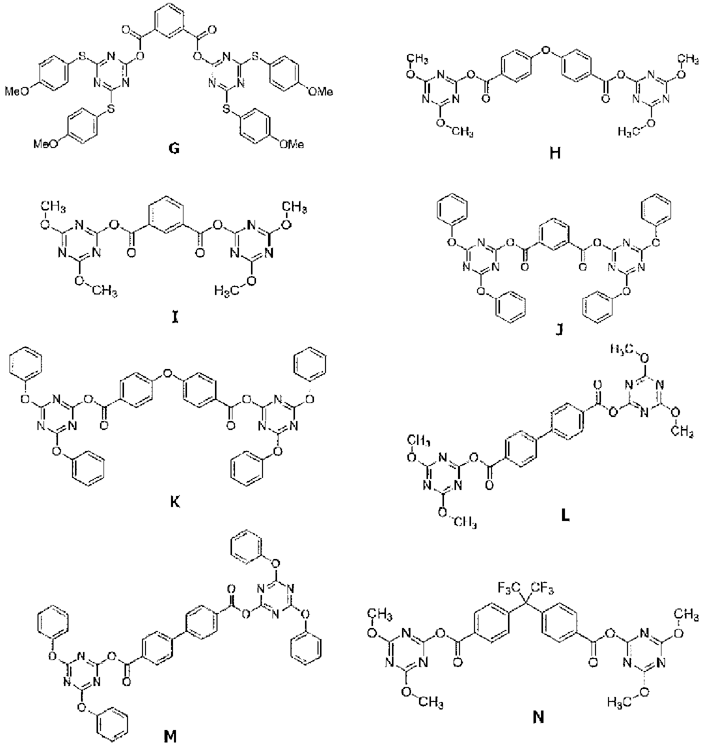

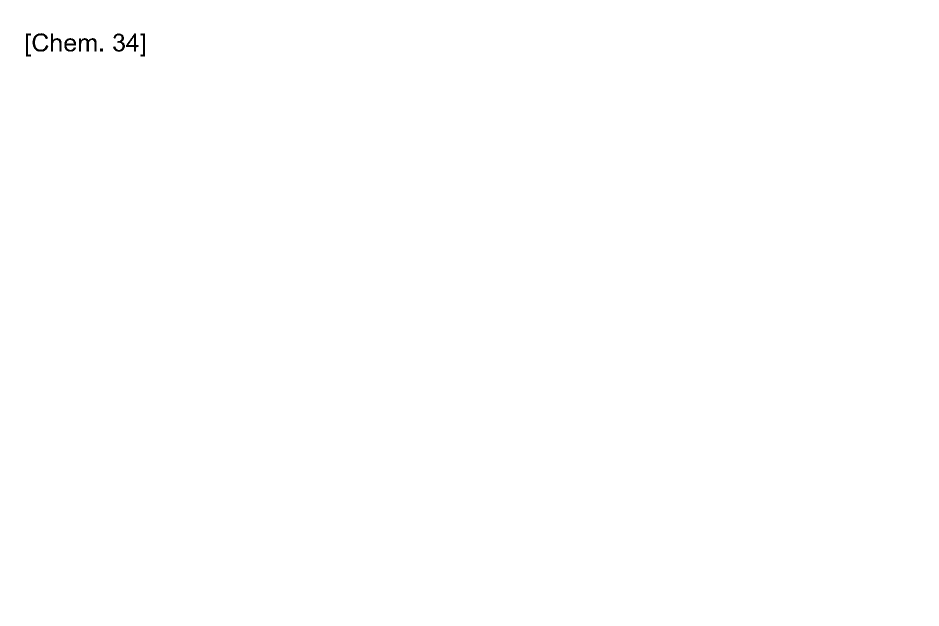

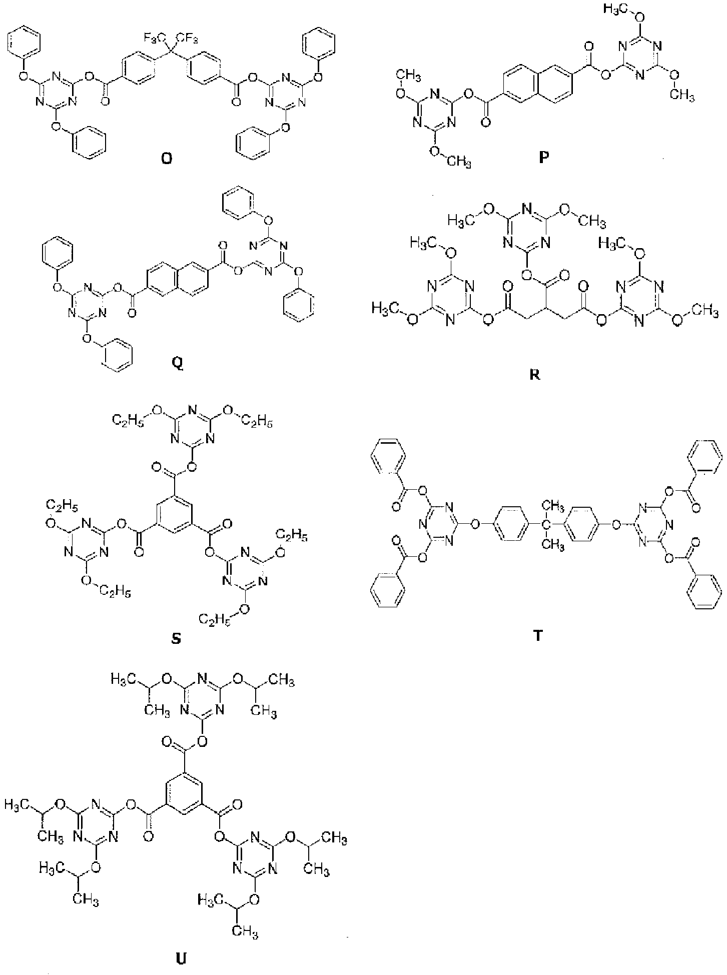

### TAB: Triacetoxybenzene

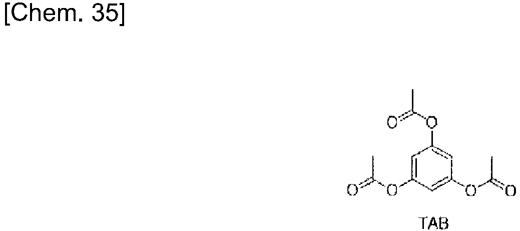

### 4. Evaluation of Multifunctional Active Ester Compound

### 4-1. Reactivity

### Example 6

The reaction temperature was measured in the same manner as in Example 1 except that Compound F was used instead of Compound A.

### Example 7

The reaction temperature was measured in the same manner as in Example 6 except that DMAP was not blended.

### Example 8

The temperature was measured in the same manner as in Example 6 except that Epoxy Compound II (CELLOXIDE 2021P available from Daicel Corporation, an epoxy compound represented by the following formula) was used instead of Epoxy Compound I (jER828 available from Mitsubishi Chemical Corporation).

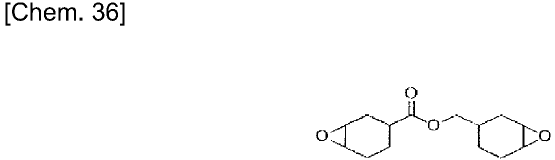

### Examples 9, 11, 15 to 25, and 27

The reaction temperatures were measured in the same manner as in Example 6 except that the compounds listed in Table 2 were used instead of Compound F.

### Examples 10 and 12

The reaction temperatures were measured in the same manner as in Example 7 except that the compounds listed in Table 2 were used instead of Compound F.

### Example 13

The reaction temperature was measured in the same manner as in Example 8 except that the compound listed in Table 2 was used instead of Compound F.

### Example 14

The reaction temperature was measured in the same manner as in Example 13 except that DMAP was not blended.

### Example 26

The reaction temperature was measured in the same manner as in Example 6 except that Epoxy Compound III (EPICLON 830 (Bis-F) available from DIC Corporation) was used instead of Epoxy Compound I (jER828 available from Mitsubishi Chemical Corporation) and Compound T was used instead of Compound F.

### Comparative Example 2

The reaction temperature was measured in the same manner as in Example 6 except that triacetoxybenzene (TAB) was used instead of Compound F.

### Comparative Example 3

The reaction temperature was measured in the same manner as in Example 13 except that triacetoxybenzene (TAB) was used instead of Compound H.

### Evaluation Results

The evaluation results for Examples 6 to 27 and Comparative Examples 2 to 3 are shown in Table 2.

**[Table 2]**

| | Reactant type | Number of functional groups of reactant | Epoxy compound | Additive | Molar ratio of ester group/epoxy group in composition | Ester compound [mg] | Epoxy compound + additive [mg] | Additive [parts by mass/100 parts by mass of epoxy compound] | Peak temperature (°C) |
|---|---|---|---|---|---|---|---|---|---|
| Example 6 | F | Multifunctional | Epoxy Compound I | DMAP | 1.0 | 125.0 | 109.1 | 2.1 | 110 |
| Example 7 | F | Multifunctional | Epoxy Compound I | - | 1.0 | 125.0 | 106.8 | 0.0 | 123 |
| Example 8 | F | Multifunctional | Epoxy Compound II | DMAP | 1.0 | 328.0 | 201.7 | 2.4 | 100 |
| Example 9 | G | Multifunctional | Epoxy Compound I | DMAP | 1.0 | 239.9 | 93.0 | 2.0 | 122 |
| Example 10 | G | Multifunctional | Epoxy Compound I | - | 1.0 | 91.6 | 35.5 | 2.0 | 141 |
| Example 11 | H | Multifunctional | Epoxy Compound I | DMAP | 1.0 | 163.3 | 103.5 | 2.0 | 129 |
| Example 12 | H | Multifunctional | Epoxy Compound I | - | 1.0 | 179.1 | 113.5 | 2.0 | 137 |
| Example 13 | H | Multifunctional | Epoxy Compound II | DMAP | 1.0 | 192.0 | 90.2 | 2.0 | 127 |
| Example 14 | H | Multifunctional | Epoxy Compound II | - | 1.0 | 220.1 | 13.4 | 0.0 | 137 |
| Example 15 | I | Multifunctional | Epoxy Compound I | DMAP | 1.0 | 106.4 | 81.4 | 2.0 | 138 |
| Example 16 | J | Multifunctional | Epoxy Compound I | DMAP | 1.0 | 128.5 | 63.1 | 2.0 | 131 |
| Example 17 | K | Multifunctional | Epoxy Compound I | DMAP | 1.0 | 84.2 | 37.2 | 2.0 | 145 |
| Example 18 | L | Multifunctional | Epoxy Compound I | DMAP | 1.0 | 101.3 | 67.4 | 2.0 | 125 |
| Example 19 | M | Multifunctional | Epoxy Compound I | DMAP | 1.0 | 153.6 | 69.4 | 2.0 | 151 |
| Example 20 | N | Multifunctional | Epoxy Compound I | DMAP | 1.0 | 142.0 | 73.3 | 2.0 | 135 |
| Example 21 | O | Multifunctional | Epoxy Compound I | DMAP | 1.0 | 192.2 | 72.5 | 2.0 | 118 |
| Example 22 | P | Multifunctional | Epoxy Compound I | DMAP | 1.0 | 99.6 | 69.8 | 2.0 | 128 |
| Example 23 | Q | Multifunctional | Epoxy Compound I | DMAP | 1.0 | 159.2 | 74.3 | 2.0 | 136 |
| Example 24 | R | Multifunctional | Epoxy Compound I | DMAP | 1.0 | 244.6 | 214.1 | 2.0 | 124 |
| Example 25 | S | Multifunctional | Epoxy Compound I | DMAP | 1.0 | 266.0 | 195.4 | 2.3 | 129 |
| Example 26 | T | Multifunctional | Epoxy Compound III | DMAP | 1.0 | 214.0 | 157.8 | 2.3 | 122 |
| Example 27 | U | Multifunctional | Epoxy Compound I | \| DMAP | 1.0 | 302.0 | 197.4 | 1.8 | 114 |
| Comparative Example 2 | TAB | Multifunctional | Epoxy Compound I | DMAP | 1.0 | 146.6 | 295.7 | 1.4 | 169 |
| Comparative Example 3 | TAB | Multifunctional | Epoxy Compound II | DMAP | 1.0 | 207 | 311 | 1.6 | 196 |

### Discussion

### (1) Differences by Type of Active Ester Compound

As shown in Table 2, it can be seen that Compounds F to U (Examples 6 to 27) have lower peak temperatures than TAB (Comparative Examples 2 and 3), and react with the epoxy compound at a lower temperature.

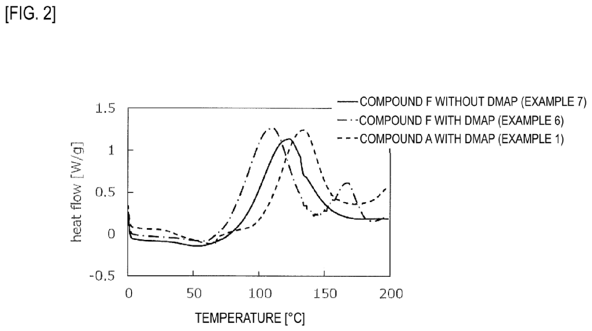

FIG. 2 is a graph showing the results of DSC measurement of the multifunctional active ester compound. For comparison, the results of DSC measurement of Compound A are also shown.
As shown in FIG. 2, it can be seen that Compound F (Example 6) reacts with the epoxy compound at a still lower reaction temperature than Compound A (Example 1). From this, it is found that Compound F exhibits still higher reactivity than Compound A.
It is also found that Compound F exhibits good reactivity even without the use of DMAP as a co-catalyst (Example 7).

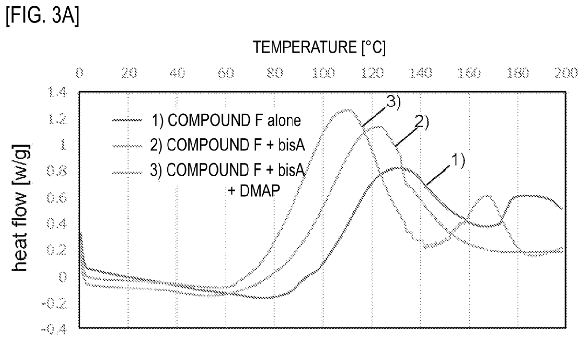

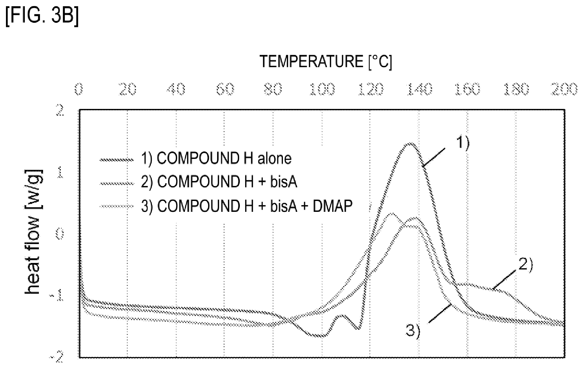

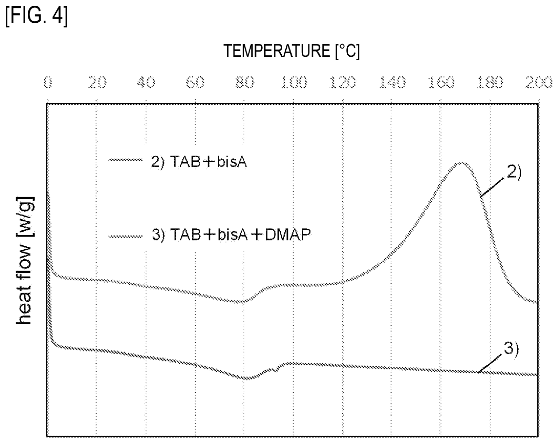

FIG. 3A is a graph showing the results of DSC measurement of Compound F, FIG. 3B is a graph showing the results of DSC measurement of Compound H, and FIG. 4 is a graph showing the results of DSC measurement of TAB. Each figure shows the results of 1) the compound alone, 2) the case where the compound was reacted with the bisphenol A-type epoxy compound, and 3) the case where the compound was reacted with the bisphenol A-type epoxy compound in the presence of DMAP.
When compared in case 3), TAB has a reaction peak near 170°C (see FIG. 4), whereas Compound H has a reaction peak near 130°C and Compound F has a reaction peak near 110°C (see FIGS. 3A and 3B), and it can be seen that both of Compounds H and F have low reaction temperatures.

### (2) Differences by Type of Epoxy Compound

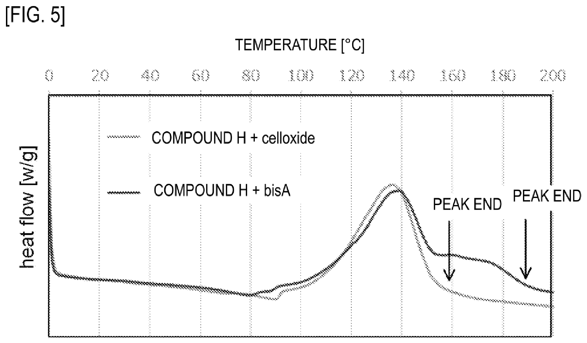

FIG. 5 is a graph showing the results of DSC measurement of Compound H (Examples 11 and 13) with different types of epoxy compounds.
As shown in FIG. 5, it can be seen that the reaction temperature when reacted with Epoxy Compound II (alicyclic epoxy compound) is almost the same as the reaction temperature when reacted with Epoxy Compound I (glycidyl-based epoxy compound). In addition, it can be seen that the position of the peak end when reacted with Epoxy Compound II (alicyclic epoxy compound) is shifted to the lower temperature side than the position of the peak end when reacted with Epoxy Compound I (glycidyl-based epoxy compound). From these results, it is found that the reaction time can be further shortened by the combination of the alicyclic epoxy compound and the active ester compound.

### 5. Examination of Compatibility

### 5-1. Preparation of Curable Composition

A vial was charged with 100 parts by mass of dimethylacetamide (DMAc), to which 10 parts by mass of Compound F (reaction temperature Tp = 110°C), 8.1 parts by mass of Epoxy Compound I, and 0.2 parts by mass of DMAP were added and dispersed in an ultrasonic wave at a temperature slightly lower than 50°C for 1 hour, but they remained undissolved.
Subsequently, the obtained composition was heated under the temperature and time conditions specified in each of Conditions 1 to 5 to form a homogeneous solution.
Condition 1: 100°C, 30 minutes
Condition 2: 100°C, 60 minutes
Condition 3: 100°C, 120 minutes
Condition 4: 100°C, 180 minutes
Condition 5: 80°C, 120 minutes
Thereafter, this solution was dried under reduced pressure at 40°C for 16 hours, and then the state after 2 hours of drying was photographed and visually observed.

### 5-2. Evaluation

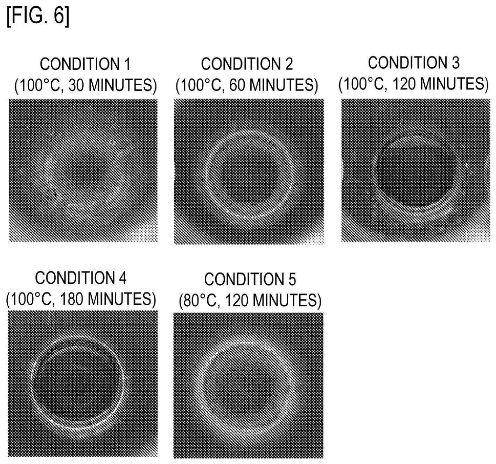

FIG. 6 illustrates photographs of the state of each curable composition after drying, taken from directly above the bottom of the vial.
As shown in FIG. 6, after 2 hours of drying, a solid precipitate was slightly observed under Conditions 1, 2, and 5, whereas no solid precipitation was observed under Conditions 3 and 4. In particular, under Condition 4, no solid precipitation was observed even after 16 hours of drying.
From these results, it is found that the compatibility between the epoxy compound and the active ester compound is further enhanced by heating at a temperature equal to or higher than the reaction temperature for a predetermined time or longer.

The present application claims priority to JP 2023-223593 filed on December 28, 2023. The contents described in the specifications and drawings of the priority application are incorporated by reference in its entirety into the specification of the present application.

### INDUSTRIAL APPLICABILITY

The reactant of the present invention can suppress the formation of a hydroxy group and be reacted with an epoxy compound even at a low reaction temperature. Therefore, the reactant of the present invention is suitable as a modifying agent or curing agent for reacting with an epoxy compound.

## Claims

1. A reactant for reacting with an epoxy compound, the reactant being an active ester compound having a structure in which an acyloxy group is directly bonded to a carbon atom constituting a 1,3,5-triazine ring.

2. The reactant according to claim 1, wherein
the reactant is a modifying agent for reacting with an epoxy compound; and
the reactant is a compound represented by Formula (1): 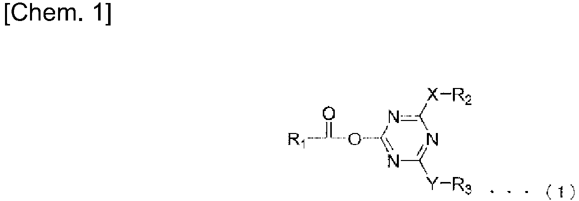
wherein in Formula (1),
R₁ to R₃ are each a monovalent organic group; and
X and Y are each an oxygen atom, -NR- (where R is a substituent), or a sulfur atom.

3. The reactant according to claim 1, wherein
the reactant is a curing agent for an epoxy compound; and
the active ester compound is a compound represented by Formula (2): 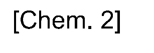 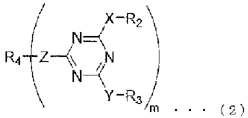
wherein in Formula (2),
R₂ and R₃ are each a monovalent organic group;
R₄ is an m-valent organic group;
m is an integer of 1 or greater;
when m is 1, at least two of X, Y, or Z are carbonyloxy bonds, and the remainder is an oxygen atom, -NR- (where R is a substituent), or a sulfur atom; and
when m is 2 or greater, at least one of X, Y, or Z is a carbonyloxy bond, and the remainder is an oxygen atom, -NR- (where R is a substituent), or a sulfur atom.

4. The reactant according to claim 3, wherein
m is an integer of 2 or greater;
Z is a carbonyloxy bond; and
X and Y are each an oxygen atom, -NR-, or a sulfur atom.

5. The reactant according to claim 3, wherein
m is an integer of 2 or greater;
X and Y are each a carbonyloxy bond; and
Z is an oxygen atom, -NR-, or a sulfur atom.

6. The reactant according to claim 1, wherein
the reactant is a curing agent for an epoxy compound; and
the active ester compound is a polymer having a structure represented by any one of Formulae (3) to (6): 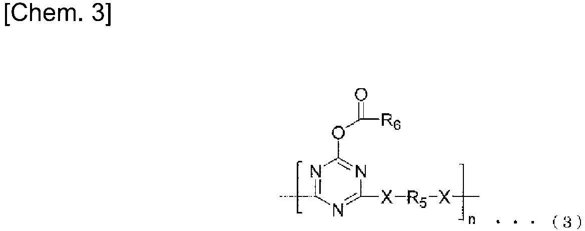 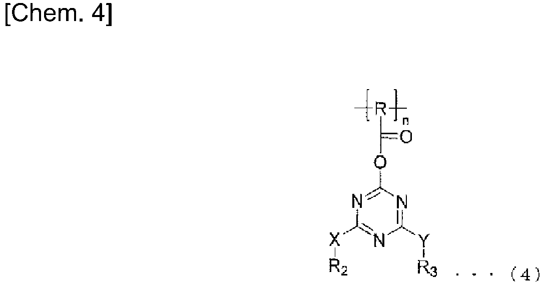
wherein in Formulae (3) and (4),
R₂, R₃, and R₆ are each a monovalent organic group;
R₅ is a divalent organic group;
R is a group constituting a repeating unit of the polymer;
X and Y are each an oxygen atom, -NR- (where R is a substituent), or a sulfur atom; and
n is an integer of 2 or greater, and 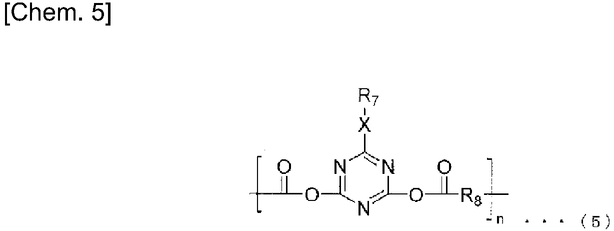 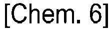 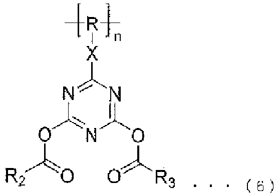
wherein in Formulae (5) and (6),
R₂, R₃, and R₇ are each a monovalent organic group;
R₈ is a divalent organic group;
R is a group constituting a repeating unit of the polymer;
X is an oxygen atom, -NR- (where R is a substituent), or a sulfur atom; and
n is an integer of 2 or greater.

7. A curable composition, comprising:
an epoxy compound; and
the reactant described in any one of claims 3 to 6.

8. The curable composition according to claim 7, wherein
the epoxy compound is an alicyclic epoxy compound.

9. A method of reacting an epoxy compound, the method comprising:
reacting an epoxy compound with the reactant described in any one of claims 1 to 6.

10. The reaction method according to claim 9, wherein
the epoxy compound is an alicyclic epoxy compound.
